# EUROPEAN PATENT SPECIFICATION

(11) **EP 4 240 296 B1**
(45) Date of publication and mention of the grant of the patent: **10.04.2024**
(21) Application number: 22705628.0
(22) Date of filing: 28.01.2022
(51) Int. Cl.: A61F 5/443, A61F 5/453, A61F 5/441, A61F 5/44

(54) **MALE FLUID COLLECTION ASSEMBLIES AND SYSTEMS**
MÄNNLICHE FLÜSSIGKEITSSAMMELANORDNUNGEN UND SYSTEME
ENSEMBLES ET SYSTÈMES DE COLLECTE DE FLUIDE MASCULIN

(43) Date of publication of application: 13.09.2023
(73) Proprietor: Purewick Corporation, El Cajon, California 92020 (US)
(72) Inventor: DAVIS, Kathleen, Atlanta, GA 30307 (US); HINE, Robert, Covington, GA 30014 (US); SZYMANIAK, Kamil, Dacula, GA 30019 (US); GRUPP, Katie, Alpharetta, GA 30009 (US)
(74) Representative: Hoffmann Eitle
(86) International application number: PCT/US2022/014285
(87) International publication number: WO 2023/146529

(56) References cited:
- WO-A1-2021/155206
- WO-A1-2022/006256
- US-A1- 2019 247 222
- US-A1- 2021 121 318

## Description

### BACKGROUND

In various circumstances, a person or animal may have limited or impaired mobility such that typical urination processes are challenging or impossible. For example, a person may experience or have a disability that impairs mobility. A person may have restricted travel conditions such as those experienced by pilots, drivers, and workers in hazardous areas. Additionally, sometimes urine collection is needed for monitoring purposes or clinical testing.

Urinary catheters, such as a Foley catheter, can be used to address some of these circumstances, such as incontinence. Unfortunately, however, urinary catheters can be uncomfortable, painful, and can lead to complications, such as infections. Additionally, bed pans, which are receptacles used for the toileting of bedridden patients, such as those in a health care facility, are sometimes used. Bed pans, however, can be prone to discomfort, spills, and other hygiene issues.

Males who suffer the most severe consequences of urinary incontinence, such as discomfort, rashes, and sores are typically elderly and often bedbound. They also require continuous assistance to maintain hygiene. Characteristics often found in these patients: they typically lay on their back, the size of the penis often decreases with age, skin rolls containing fat tissue cause the penis to recede, often pointing upward while in a laying position, patients have difficulty reaching the penis and manipulating devices. A urine capture device should be designed with reference to these characteristics.

Available solutions are typically for use while standing up (such as cups and funnels), with a urine discharge port opposite to the distal end of the penis. Other designs such as condom-style catheters are difficult for patients to manipulate, too often they are dimensionally incompatible; and they do not stay on reliably.

Examples of patent publications of condom-type male urinary incontinence devices with a drain conduit arranged on the longitudinal axis of the device can be found in US2019/0247222A1, US2021/121318A1, WO2021/2021/155206A1 and WO2022/006256 (published 06 January 2022). See US 5,300,052 for a device in which the drain outlet is inclined to the length axis of the device and, albeit close to, not at the lowest point of the urine reservoir within the device.

US2019/0247222A1 discloses a urine collection device including a collection member that extends from a proximal end to a distal end. At the proximal end is an opening to receive a penis into an internal cavity of the collection member. At the distal end is an outlet conduit.

Thus, there is a need for a device capable of collecting urine from a person or animal, particularly a male, comfortably and with minimal contamination of the user and/or the surrounding area.

### SUMMARY

The invention is defined in claim 1, in which the features of the precharacterising portion are disclosed in US2019/0247222A1. The dependent claims are directed to optional features and preferred embodiments

Disclosed herein are male fluid collection assemblies, systems including the same, methods of manufacturing the same, and methods of using the same. A fluid collection assembly is disclosed. The fluid collection assembly includes a sheath. The sheath includes a fluid impermeable barrier including a proximal region and a distal region extending from the proximal region. The proximal region exhibits a first width and the distal region exhibiting a second width that is less than the first width. The proximal region defines an opening and the distal region defines a fluid outlet. The fluid impermeable barrier at least defines a chamber. The sheath also includes at least one porous material disposed in the chamber. The fluid collection assembly also includes a base secured to or configured to be secured to the proximal region of the sheath. The base is configured to be attached to skin surrounding a penis. The base defines an aperture that corresponds to the opening of the sheath.

A system is disclosed. The system includes a fluid collection assembly. The fluid collection assembly includes a sheath. The sheath includes a fluid impermeable barrier including a proximal region and a distal region extending from the proximal region. The proximal region exhibits a first width and the distal region exhibiting a second width that is less than the first width. The proximal region defines an opening and the distal region defines a fluid outlet. The fluid impermeable barrier at least defines a chamber. The sheath also includes at least one porous material disposed in the chamber. The fluid collection assembly also includes a base secured to or configured to be secured to the proximal region of the sheath. The base is configured to be attached to skin surrounding a penis. The base defines an aperture that corresponds to the opening of the sheath. The system also includes a vacuum source configured to apply a vacuum force, a fluid storage container, and at least one conduit connected to the outlet and in fluid communication with the vacuum source and the fluid storage container.

A method of manufacturing a fluid collection assembly is disclosed (but not claimed). The method includes attaching a base to a fluid impermeable barrier of a sheath. The sheath including a fluid impermeable barrier including a proximal region and a distal region extending from the proximal region. The proximal region exhibits a first width and the distal region exhibiting a second width that is less than the first width. The proximal region defines an opening and the distal region defines a fluid outlet. The fluid impermeable barrier at least defines a chamber. The base is configured to be attached to skin surrounding a penis. The base defines an aperture that corresponds to the opening of the sheath after attaching the base to the fluid impermeable barrier. The method also includes disposing at least one porous material in the chamber.

A method of using a system to collect bodily fluids from an individual is disclosed (but not claimed). The method includes attaching a base to skin surrounding a penis. The base secured to or configured to be secured to a sheath. The sheath includes a fluid impermeable barrier including a proximal region and a distal region extending from the proximal region. The proximal region exhibits a first width and the distal region exhibiting a second width that is less than the first width. The proximal region defines an opening and the distal region defines a fluid outlet. The fluid impermeable barrier at least defines a chamber. The sheath also includes at least one porous material disposed in the chamber.

Features from any of the embodiments of the invention disclosed hereinbelow may be used in combination with one another, without limitation. In addition, other features and advantages of the present invention will become apparent to those of ordinary skill in the art through consideration of the following detailed description and the accompanying drawings.

### BRIEF DESCRIPTION OF THE DRAWINGS

The drawings illustrate several embodiments of the present invention, wherein identical reference numerals refer to identical or similar elements or features in different views or embodiments shown in the drawings.
**FIGS. 1A** and **1B** are isometric top and bottom views, respectively, of a fluid collection assembly, according to an embodiment.
**FIGS. 1C** and **1D** are cross-sectional schematics of the fluid collection assembly taken along planes C-C and D-D, respectively.
**FIG. 1E** is an exploded isometric view of the the fluid collection assembly shown in **FIGS. 1A** and **1B****.**
**FIG. 1F** is a cross-sectional view of the port taken along plane 1F-1F shown in **FIG. 1A****.**
**FIG. 1G** is a cross-sectional schematic of a porous material.
**FIG. 1H** is a top plan view of the base.
**FIG.** 1I is a cross-sectional schematic of the base.
**FIG. 2** is a cross-sectional schematic view of a fluid collection assembly that includes a first panel and a second panel integrally formed together.
**FIGS. 3A** and **3B** are cross-sectional schematics illustrating how substantially similar fluid collection assemblies may be used with a buried and non-buried penis.
**FIG. 4** is a block diagram of a system for fluid collection.
**FIG. 5** is a flow diagram of a method to collect fluid.
**FIG. 6** is a flow diagram of a method to manufacture a fluid collection assembly.

### DETAILED DESCRIPTION

Disclosed herein are male fluid collection assemblies, systems including the same, methods of manufacturing the same, and methods of using the same. An example fluid collection assembly includes a sheath and a base. The sheath includes a fluid impermeable barrier having a proximal region and a distal region extending from the proximal region. The proximal region exhibits a first width and the distal region exhibits a second width that is less than the first width. The proximal region defines an opening and the distal region defines a fluid outlet. The fluid impermeable barrier defines a chamber. The sheath also includes at least one porous material disposed in the chamber. The base may be attached (*e.g*., permanently attached) to the sheath or the base may be configured to be secured to the sheath at some time period in the future. The base defines an aperture that is aligned with the opening when the base is attached to the sheath. The base is configured to be secured to a region about a penis of an individual with the aperture positioned over the penis.

A method of using the fluid collection assembly can include securing the base to the region about the penis of an individual. The base is positioned on the individual such that the penis extends through *(e.g.,* the penis is not buried) or is adjacent to (*e.g.*, the penis is buried) the aperture defined by the base. If sheath is not already attached to the base, the sheath may also be attached to the base. For example, the sheath may be attached to the base before, during, or after securing the base to the region about the penis. After the base is secured to the region about the penis and the sheath is attached to the base, the individual may discharge bodily fluids from the penis. The bodily fluids may include urine or sweat. The bodily fluids enter the chamber of the sheath. The porous material may receive at least some of the bodily fluids that enter the chamber and direct the bodily fluids towards the fluid outlet. The method may include removing the bodily fluids from the chamber through the fluid outlet, for instance, when a vacuum force is applied to the outlet via a vacuum source that is in fluid communication with the chamber. Although the fluid collection assemblies disclosed herein are discussed as being male fluid collection assemblies that are used with penises, it is noted that the fluid collection assemblies may be used to collect bodily fluids from females.

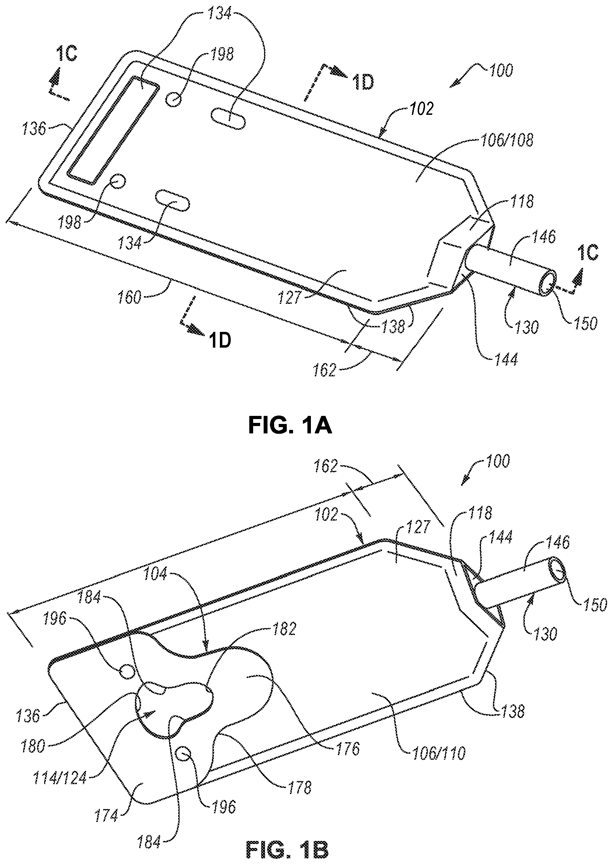

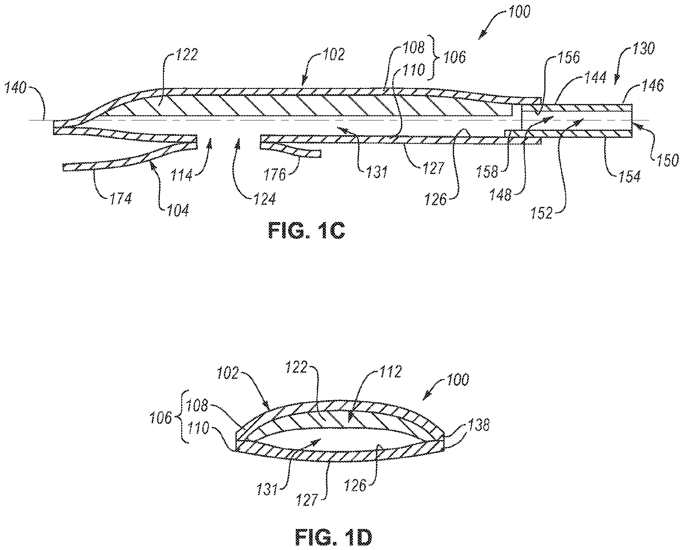

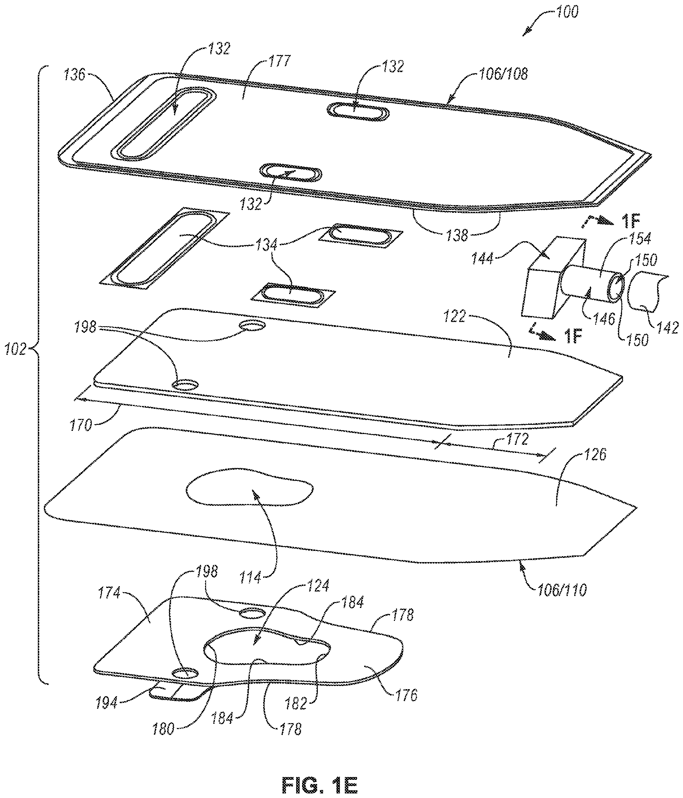

**FIGS. 1A** and **1B** are isometric top and bottom views, respectively, of a fluid collection assembly 100, according to an embodiment. **FIGS. 1C** and **1D** are cross-sectional schematics of the fluid collection assembly 100 taken along planes C-C and D-D, respectively. **FIG. 1E** is an exploded view of the fluid collection assembly 100. The fluid collection assembly 100 includes a sheath 102 and a base 104. According to the invention, the sheath 102 includes a fluid impermeable barrier 106 that is at least partially formed from a first panel 108 attached to a second panel 110. In an embodiment, as illustrated, the first panel 108 and the second panel 110 are distinct sheets. The fluid impermeable barrier 106 also defines a chamber 112 between the first panel 108 and the second panel, an opening 114, and a fluid outlet 118. The sheath 102 also includes at least one porous material 122 disposed in the chamber 112. The base 104 includes an aperture 124. The base 104 is attached to the proximal region 160 of the fluid impermeable barrier 106 such that the aperture 124 is aligned with the opening 114.

The inner surfaces 126 of the fluid impermeable barrier 106 (*e.g.*, inner surfaces of the first and second panels 108, 110) at least partially defines the chamber 112 within the fluid collection assembly 100. The fluid impermeable barrier 106 temporarily stores the bodily fluids in the chamber 112.

The fluid impermeable barrier 106 may be formed of any suitable fluid impermeable material(s), such as a fluid impermeable polymer (*e.g*., silicone, polypropylene, polyethylene, polyethylene terephthalate, polyurethane, polyethylene, polyvinyl chloride, a polycarbonate, etc.), a metal film, natural rubber, another suitable material, or combinations thereof. As such, the fluid impermeable barrier 106 substantially prevents the bodily fluids from passing through the fluid impermeable barrier 106. In an example, the fluid impermeable barrier 106 may be air permeable and fluid impermeable thus preventing leaks while allowing air flow through the chamber 112 when a vacuum force is applied thereto (*i.e.*, the chamber 112 remains at about atmospheric pressure thereby preventing the vacuum force from causing a hickie or kinking the conduit 142). In such an example, the fluid impermeable barrier 106 may be formed of a hydrophobic material that defines a plurality of pores. At least one or more portions of at least an outer surface 127 of the fluid impermeable barrier 106 may be formed from a soft and/or smooth material, thereby reducing chaffing.

The fluid impermeable barrier 106 can be formed from polyurethane, such as formed only from polyurethane. It has been found in clinical trials that forming the fluid impermeable barrier 106 from polyurethane improves the functionality of the fluid collection assembly 100. For example, the fluid impermeable barrier 106 may exhibit a flexibility when formed from polyurethane that is greater than when the fluid impermeable barrier 106 is formed from another material. The increased flexibility of the fluid impermeable barrier 106 formed from the polyurethane makes it easier to attach the fluid collection assembly 100 to the individual and maintain the fluid collection assembly 100 attached to the individual. The increased flexibility of the fluid impermeable barrier 106 formed from the polyurethane also help allow the fluid collection assembly 100 to conform to the individual and/or fit underneath the clothing of the individual thereby increasing patient comfort. The increased flexibility of the fluid impermeable barrier 106 prevents or at least inhibits corners formed in the fluid impermeable barrier 106, if present, from uncomfortably pressing into the individual using the fluid collection assembly 100 thereby making the fluid collection assembly 100 more comfortable to use. The polyurethane may also remain flexible when welded or otherwise have seals formed therein. It has also been found that individuals using the fluid collection assembly 100 describe the fluid impermeable barrier 106 formed from polyurethane as being more smooth than when the fluid impermeable barrier 106 is formed from other materials. The smoother feel of the fluid impermeable barrier 106 formed from polyurethane makes wearing the fluid collection assembly 100 more comfortable. It has also been found that forming the fluid impermeable barrier 106 from the polyurethane allows the fluid impermeable barrier 106 to be bent (*e.g*., crumpled, wrinkled, etc.) more quietly than if the fluid impermeable barrier 106 was formed from another material. The improved quietness of the fluid impermeable barrier 106 allows the fluid collection assembly 100 to be used more discreetly. For example, movement of an individual using the fluid collection assembly 100 is likely to cause the fluid impermeable barrier 106 to bend. Forming the fluid impermeable barrier 106 from polyurethane allows the individual to move more substantially without the fluid impermeable barrier 106 generating noise. Finally, it has been found that the fluid impermeable barrier 106 that includes polyurethane is able to be welded to a variety of materials thereby facilitating attachment of the base 104, the vents 134, and the port 130 to the fluid impermeable barrier 106.

At least one of the first panel 108 or the second panel 110 can be formed from an at least partially transparent fluid impermeable material, such as polyethylene, polypropylene, polyurethane, polycarbonate, or polyvinyl chloride. Forming at least one of the first panel 108 or the second panel 110 from an at least partially transparent fluid impermeable material allows a person (*e.g.*, medical practitioner) to examiner the penis. In some embodiments, both the first panel 108 and the second panel 110 are formed from at least partially transparent fluid impermeable material. For example, some conventional fluid collection assemblies that include a sheath and a base may allow the sheath to be reversibly detached from the base after the base is secured to the region about the penis. Detaching the sheath from the base allows the person to examine the penis. However, configuring the sheath to be detachable from the base may allow leaks between the sheath and the base. As previously discussed, the sheath 102 may be permanently attached to the base 104, which substantially prevents leaks between the sheath 102 and the base 104 when the base 104 is appropriately attached to the sheath 102 (*e.g*., no wrinkles were allowed to form between the sheath 102 and base 104). Selecting at least one of the first panel 108 or the second panel 110 to be formed from an at least partially transparent impermeable material allows the penis to be examined without detaching the entire fluid collection assembly 100 from the region about the penis. For example, the chamber 112 may include a penis receiving area 131 that is configured to receive the penis of the individual when the penis extends into the chamber 112. The penis receiving area 131 may be defined by at least the porous material 122 and at least a portion of the at least partially transparent material of the first panel 108 and/or the second panel 110. In other words, the porous material 122 is positioned in the chamber 112 such that the porous material is not positioned between the penis and at least a portion of the transparent portion of the first panel 108 and/or second panel 110 when the penis is inserted into the chamber 112 through the opening 114. The porous material 122 is generally not transparent and, thus, the portion of the at least partially transparent material of the first panel 108 and/or the second panel 110 that defines the penis receiving area 131 forms a window which allows the person to view into the penis receiving area 131 and examine the penis.

The second panel 110 can be at least partially formed from the at least partially transparent material and forms the window that allows the person to view into the penis receiving area 131. Further, the porous material 122 is positioned between the penis receiving area 131 and at least a portion of the first panel 108. Such an embodiment may help maintain the dignity of the individual using the fluid collection assembly 100. For example, during use, the second panel 110 is generally adjacent to the individual, such as adjacent to the thighs and/or perineum. Thus, the second panel 110 is generally obscured during use and a person cannot view the penis without first lifting the sheath 102 away from the individual. Meanwhile, the first panel 108 may face away from the individual and be more easily viewable than the second panel 110. However, a person (*e.g*., a passerby, a visitor, etc.) cannot view the penis through the first panel 108 because the porous material 122 is not transparent and/or the first panel 108 is formed from a non-transparent material. Thus, in such an embodiment, the first panel 108 and/or the porous material 122 prevent person(s) from viewing the penis unless such examination is necessary, thereby preserving the dignity of the individual using the fluid collection assembly 100. In an embodiment, the first panel 108 is formed from the at least partially transparent material and forms the window that allows the person to view into the penis receiving area 131. Further, the porous material 122 is positioned between the penis receiving area 131 and at least a portion of the second panel 110. In such an embodiment, the person does not need to perform the additional act of lifting the sheath 102 to view into the penis receiving area 131 but may not maintain the dignity of the individual using the fluid collection assembly 100 since passersby may also view into the penis receiving area 131.

As previously discussed, at least a portion of the first panel 108 and at least a portion of the second panel 110 are attached together. In the embodiment shown, the first and second panels 108, 110 are attached together along at least a portion of the outer edges thereof (*e.g*., the top and lateral edges 136, 138). In such an embodiment, the first and second panels 108, 110 are attached using any suitable technique, such as with an adhesive, sewing, heat sealing, impulse heating, direct heating, radio frequency ("RF") welding, ultrasonic ("US") welding, or any other technique. In a particular embodiment, the first and second panels 108 are attached together using impulse heating since impulse heating is quick and is effective at attaching polyurethane panels together. As will be discussed in more detail below, forming the fluid impermeable barrier 106 from the first panel 108 and the second panel 110 may improve the rate of manufacturing the fluid collection assembly 100, especially when the first panel 108 and the second panel 110 are attached together using a non-sewing technique.

The fluid impermeable barrier 106 can define one or more orifices 132 extending therethrough. The sheath 102 can include one or more vents 134 attached to the fluid impermeable barrier 106 that extend across the one or more orifices 132. The vents 134 are configured to allow air to flow therethrough while preventing water (a major constituent of bodily fluids) from flowing therethrough. The vents 134 facilitate air flow through the chamber 112. For example, as previously discussed, a vacuum may be provided to the chamber 112 from a vacuum source. The vacuum may pull air through the vents 134, thereby allowing air flow from the vents 134 to the fluid outlet 118. The air flow from the vents 134 helps move bodily fluids towards the fluid outlet 118. The vents 134 also prevent the vacuum applied to the chamber 112 from bursting small superficial blood vessels (*e.g*., cause a hickie) in or otherwise damage the penis or the area about the penis.

The vents 134 may include a porous hydrophobic material. The pores defined by the porous hydrophobic material may be interconnected thereby allowing air to flow through the vents 134. The hydrophobic properties of the porous hydrophobic material may prevent water from flowing through the vents 134. In an example, the average size of the pores (e.g., the average maximum lateral dimension of the pores) of the porous hydrophobic material may be selected to be about 1 µm or greater, about 5 µm or greater, about 10 µm or greater, about 20 µm or greater, about 30 µm or greater, about 40 µm or greater, about 50 µm or greater, about 60 µm or greater, about 80 µm or greater, about 100 µm or greater, about 125 µm or greater, about 150 µm or greater, about 175 µm or greater, about 200 µm or greater, about 250 µm or greater, about 300 µm or greater, about 400 µm or greater, about 500 µm or greater, about 1 mm or less, about 750 µm or less, about 500 µm or less, or in ranges of about 1 µm to about 10 µm, about 5 µm to about 20 µm, about 10 µm to about 30 µm, about 20 µm to about 40 µm, about 30 µm to about 50 µm, about 40 µm to about 60 µm, about 50 µm to about 80 µm, about 60 µm to about 100 µm, about 80 µm to about 125 µm, about 100 µm to about 150 µm, about 125 µm to about 175 µm, about 150 µm to about 200 µm, about 175 µm to about 250 µm, about 200 µm to about 300 µm, about 250 µm to about 400 µm, about 300 µm to about 500 µm, about 400 µm to about 750 µm, or about 500 µm to about 1 mm. In an example, the porous hydrophobic material may exhibit a contact angle with water that is greater than 90°, such as in ranges of about 90° to about 110°, about 100° to about 120°, about 110° to about 130°, about 120° to about 140°, about 130° to about 150°, about 140° to about 160°, about 150° to about 170°, or about 160° to 180°. Generally, the size of the pores is dependent on the hydrophobicity of the porous hydrophobic material and *vice versa.* For example, increasing the hydrophobicity of the porous hydrophobic material allows the size of the pores to be increased.

In an embodiment, the vents 134 may include a porous polytetrafluoroethylene ("PTFE") layer. It is currently believed that the porous PTFE layer is exceptionally effectively at allowing air to flow therethrough while preventing water from flow therethrough even when the porous PTFE layer is exposed to acidic bodily fluids. However, porous PTFE is difficult to weld to other materials, such as polyurethane. As such, the porous PTFE layer may be attached to a substrate. The substrate may include a porous material or define one or more passageways therethrough. The substrate may be selected to be easily attached to the porous PTFE layer and the fluid impermeable barrier 106. For example, the substrate may be selected to be formed from polyvinyl chloride since polyvinyl chloride may be easily attached to the porous PTFE layer and attached to the fluid impermeable barrier 106.

In an embodiment, the orifices 132 are formed in and the vents 134 are attached to the first panel 108 of the fluid impermeable barrier 106. In such an embodiment, the orifices 132 and the vents 134 are unlikely to be covered by and contact the skin of the individual. Covering the orifices 132 and the vents 134 with the skin may prevent or at least inhibit air flow through the portions of the orifices 132 and the vents 134 at are covered by the skin. Further, an individual may find the vents 134 contacting the skin to be uncomfortable and, thus, disposing the vents 134 on the first panel 108 may make the fluid collection assembly 100 more comfortable to wear. In an embodiment, the vents 134 are attached to an interior of the first panel 108 to prevent the edges of the vents 134 from rubbing or otherwise irritating the skin of the individual.

In an embodiment, as illustrated, at least one of the orifices 132 is formed in and at least one of the vents 134 is attached to a portion of the fluid impermeable barrier 106 that is at or near to top edge 136 of the fluid impermeable barrier 106. The top edge 136 of the fluid impermeable barrier 106 may include an edge of the fluid impermeable barrier 106 that is above the opening 114 when the individual is standing and the fluid collection assembly 100 is allowed to freely hang from the pubic region of the individual. Forming the orifices 132 and attaching the vents 134 at or near the top edge 136 causes air flowing through the vents 134 to pass over the penis (thereby keeping the penis dry) and inhibits pooling of the bodily fluids near the top edge 136. In an embodiment, as illustrated, at least one of the orifices 132 is formed in and at least one of the vents 134 is attached to a portion of the fluid impermeable barrier 106 that is at or near to a lateral edge 138 of the fluid impermeable barrier 106. The lateral edge 138 of the fluid impermeable barrier 106 may include an edge of the fluid impermeable barrier 106 that extends from or near the top edge 136. Generally, air preferentially flows in a path extending from the vents 134 towards the fluid outlet 118. When the vents 134 are spaced from the lateral edges 138 and/or at or near the top edge 136, the preferential air flow from these vents 134 may cause at least some of the portions of the chamber 112 adjacent to the lateral edges 138 to have minimal air flow therethrough. Positioning the orifices 132 and the vents 134 at or near the lateral edges 138 may increase the air flow through the portions of the chamber 112 adjacent to the lateral edges 138 thereby preventing or at least inhibiting pooling of bodily fluids in such portions of the chamber 112.

The orifices 132 and the vents 134 may exhibit an elongated shape. For example, the orifices 132 and the vents 134 at or near the top edge 136 may exhibit an elongated shape that is generally parallel to the top edge 136 and/or generally perpendicular to a longitudinal axis 140 of the fluid collection assembly 100. In such an example, the elongated shape of the orifices 132 and the vents 134 cause the air to preferentially flow through a greater percentage of the chamber 112 than if the orifices 132 and the vents 134 did not exhibit an elongated shape and/or were oriented differently. In an example, the orifices 132 and the vents 134 at or near the lateral edge 138 may exhibit an elongated shape that is generally parallel to the lateral edge 138 and/or generally parallel to the longitudinal axis 140 of the fluid collection assembly 100. In such an example, the elongated shape of the orifices 132 and the vents 134 may cause the air to preferentially flow through a greater percentage of the chamber 112 (*e.g*., the portions of the chamber 112 adjacent to the lateral edges 138) than if the orifices 132 and the vents 134 did not exhibit an elongated shape and/or were oriented differently.

The opening 114 defined by the fluid impermeable barrier 106 provides an ingress route for fluids to enter the chamber 112 when the penis is a buried penis and allow the penis to enter the chamber 112 (*e.g*., the penis receiving area 131) when the penis is not buried. The opening 114 may be defined by the fluid impermeable barrier 106 (*e.g.*, an inner edge of the fluid impermeable barrier 106). For example, the opening 114 is formed in and extends through the fluid impermeable barrier 106, from the outer surface 127 to the inner surface 126, thereby enabling bodily fluids to enter the chamber 112 from outside of the fluid collection assembly 100.

The second panel 110 can define the entirety of the opening 114. For example, the opening 114 is a cutout defined by the second panel 110 that is spaced from the outer periphery (*e.g*., edges) of the second panel 110. In such an example, the second panel 110 may exhibit a shape that substantially corresponds to the shape of the first panel 108 which may facilitate attaching the first panel 108 to the second panel 110 along the outer periphery thereof. It also allows the first panel 108 and the second panel 110 to lie substantially flat when the penis is not in the chamber 112 and the sheath 102 is lying on a flat surface. The ability of the first and second panels 108, 110 to lie substantially flat may make wearing the fluid collection assembly 100 more discrete and inhibit pooling of bodily fluids against the individual. However, in some embodiments, the opening 114 is not spaced from the outer periphery of the second panel 110. In such embodiments, the opening 114 may be a cutout extending inwardly from at least one outer periphery of the second panel 110. Other examples of forming the opening 114 in the second panel 110 are disclosed in WO 2022/006256, International Patent Application No. PCT/US2021/039866 filed on June 30, 2021.

As previously discussed, the fluid impermeable barrier 106 includes a proximal region 160 and a distal region 162 extending from the proximal region 160, for example, to the fluid outlet 118 (*e.g.*, to the port 130). The proximal region 160 defines the opening 114 and the distal region 162 defines the fluid outlet 118. Both the proximal region 160 and the distal region 162 define the chamber 112.

The fluid impermeable barrier 106 can exhibit a generally bullet shape. As used, herein, the fluid impermeable barrier 106 exhibits the generally bullet shape when the proximal region 160 of the fluid impermeable barrier 106 exhibits a substantially constant first width and the distal region 162 of the fluid impermeable barrier 106 exhibits a second width that is less than the first width. The first and second widths may be measured perpendicularly to the longitudinal axis 140 and may be greater than a thickness of the sheath 102 when the sheath 102 lies on a flat surface. The generally bullet shape of the fluid impermeable barrier 106 facilitates operation of the fluid collection assembly 100 while making the sheath 102 more comfortable. For example, the substantially constant first width maintains contact with the upper portions of the thighs of the individual thereby maintaining the position of the fluid collection assembly 100. Also, the substantially constant first width makes it easier for the penis to become erect since the substantially constant first width inhibits the sheath 102 falling between the thighs of the individual and the erect penis from lifting the portions of the sheath 102 that fell between the thighs. The decreased second width makes the sheath 102 thinner thereby allowing the portions of the sheath 102 that are less likely to receive the penis to drop between the thighs of the individual. This drop allows gravity to assist in moving the bodily fluids towards the fluid outlet 118. The decreased second width also funnels the bodily fluids towards the fluid outlet 118 and the port 130.

The second width of the distal region 162 may vary (*e.g*., decrease) along a length of the distal region 162 measured perpendicularly to the longitudinal axis 140. In an example, the second width of the distal region 162 may decrease at a constant rate from the proximal region 160 towards (*e.g*., to) the fluid outlet 118. The constant rate at which the second width decreases causes the lateral edges 138 of the fluid impermeable barrier 106 that defines the distal region 162 to be straight. The straight lateral edges 138 may form corners at the intersection between the proximal and distal regions 160, 162 that may uncomfortably press into the individual. In an example, the second width of the distal region 162 may decrease at a variable (*e.g*., increasing) rate from the proximal region 160 towards (*e.g*., to) the fluid outlet 118. The variable rate at which the second width decreases causes the lateral edges 138 of the fluid impermeable barrier 106 that defines the distal region 162 to be curved, such as convexly curved. The curved lateral edges 138 may prevent the formation of corners that may press into the individual thereby making the fluid collection assembly 100 more comfortable than if the lateral edges 138 of the distal region 162 were straight. However, it is noted that the air flow through the distal region 162 may be more uniform when the lateral edges 138 of the distal region 162 are straight instead of curved.

It is noted that the fluid impermeable barrier 106 may exhibit a shape other than the generally bullet shape. In an example, the fluid impermeable barrier 106 may exhibit a generally rectangular shape, as discussed in more detail in aforementioned WO 2022/006256. In an example, the fluid impermeable barrier 106 may exhibit a generally triangular shape, a semi-oval shape, or any other suitable shape.

The fluid impermeable barrier 106 defines a fluid outlet 118. The fluid outlet 118 is formed from portions of the first panel 108 and the second panel 110 that are not attached together. In an embodiment, not shown, the fluid outlet 118 is configured to be directly attached to a conduit 142. In such an embodiment, the conduit 142 may be at least partially disposed in the chamber 112 or otherwise in fluid communication with the chamber 112 through the fluid outlet 118. The fluid outlet 118 may be sized and shaped to form an at least substantially fluid tight seal against the conduit 142, thereby substantially preventing the bodily fluids from escaping the chamber 112. Attaching the conduit 142 to the fluid outlet 118 may prevent leaks and may prevent the conduit 142 from inadvertently becoming detached from the fluid outlet 118. In an embodiment, as shown, the fluid outlet 118 is configured to be indirectly attached to the conduit 142. In such an embodiment, the sheath 102 may include a port 130 that is directly attached to the fluid impermeable barrier 106 and configured to be attached to the conduit 142.

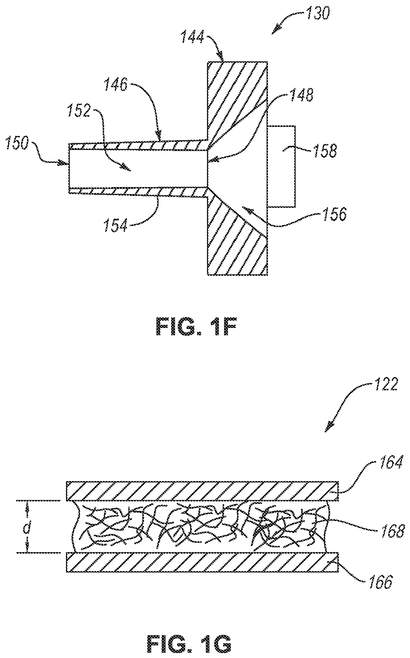

**FIG. 1F** is a cross-sectional view of the port 130 taken along plane 1F-1F shown in **FIG. 1E****.** The port 130 includes a first part 144 and a second part 146. The first part 144 is configured to be attached to the fluid impermeable barrier 106 and, optionally, to be at least partially disposed in the chamber 112. The second part 146 is configured to attach to the conduit 142. The first part 144 defines an inlet 148 and the second part 146 defines an outlet 150 of the port 130 that is downstream from the inlet 148. The port 130 also defines a channel 152 extending from the inlet 148 to the outlet 150. It is noted that the inlet 148 refers to the inlet of the channel 152 and not necessarily the inlet of the port 130. In an example, the inlet 148 may be the inlet of both the port 130 and the channel 152 when the port 130 does not include a sink 156. In an example, as illustrated, the inlet 148 may not be the inlet of the port 130 when the port 130 includes sink 156 since the sink 156 forms the inlet of the port 130.

The first part 144 of the port 130 is attached to the fluid impermeable barrier 106 using any suitable technique. In an embodiment, the first part 144 is disposed between the first and second panels 108, 110 before attaching the first part 144 to the fluid impermeable barrier 106. In such an embodiment, the first part 144 abuts and is attached to the inner surface 126 of the fluid impermeable barrier 106. In an embodiment, the first part 144 is attached to the fluid impermeable barrier 106 using at least one of an adhesive, impulse heating, direct heating, US welding, RF welding, any other attachment technique disclosed herein, or another other suitable attachment technique.

At least the second part 146 can exhibit a rigidity that is greater than the fluid impermeable barrier 106. The second part 146 may exhibit a rigidity that is greater than the fluid impermeable barrier 106 because at least one of second part 146 exhibits a thickness that is greater than the fluid impermeable barrier 106 or at least a portion of the second part 146 is formed from a material exhibiting a Young's modulus (*i.e*., modulus of elasticity) that is greater than a Young's modulus of a material forming at least a portion of the fluid impermeable barrier 106. The increased rigidity of the second part 146 relative to the fluid impermeable barrier 106 allows the second part 146 to be attached to the conduit 142 using techniques that may be difficult or impossible to do when the conduit 142 is directly attached to the fluid impermeable barrier 106. In an example, the conduit 142 may be attached to the second part 146 using an interference fit which may be difficult or impossible to do when the conduit 142 is directly attached to the fluid impermeable barrier 106. In such an example, a surface of the second part 146 that is configured to abut the conduit 142 is tapered. The tapered surface of the second part 146 may make inserting the second part 146 into the conduit 142 easier when the second part 146 forms a male connector and may make receiving the conduit 142 into the second part 146 easier when the second part 146 forms a female connector. The tapered surface of the second part 146 also allows the strength of the interference fit between the second part 146 and the conduit 142 to be controlled by controlling how much the second part 146 is inserted into the conduit 142 or how much the conduit 142 is inserted into the second part 146. In an example, not shown, a surface of the second part 146 is configured to be threadedly attached to the conduit 142 which may be difficult or impossible to do when the conduit 142 is directly attached to the fluid impermeable barrier 106. In such an example, the second part 146 may include one or more helically extending ridges extending from a surface of the second part 146 that contacts or is closest to the conduit 142. It is noted that the surface of the second part 146 that includes the threads and/or the threads may be tapered. In an example, not shown, a surface of the second part 146 that contacts or is closest to the conduit 142 may include circumferentially extending ridges. It is noted that the surface of the second part 146 that includes the ridges and/or the ridges may be tapered.

At least the second part 146 can exhibit a rigidity that is greater than the conduit 142. The second part 146 may exhibit a rigidity that is greater than the conduit 142 because at least one of the second part 146 exhibits a thickness that is greater than the conduit 142 or at least a portion of the second part 146 is formed from a material exhibiting a Young's modulus that is greater than a Young's modulus of a material forming at least a portion of the conduit 142. The increased rigidity of the second part 146 prevents collapse of the channel 152. For example, it has been found that, in certain circumstances, directly attaching the conduit 142 to the fluid impermeable barrier 106 may cause the portions of the conduit 142 attached to the fluid impermeable barrier 106 to at least partially collapse when a strong vacuum is applied to the chamber 112. However, the increased rigidity of the port 130 prevents such collapse.

The first part 144 can exhibit a rigidity that is greater than the fluid impermeable barrier 106 and/or the conduit 142. For example, the first part 144 may exhibit a rigidity that is comparable to the second part 146. The increased rigidity of the first part 144 prevents collapse of the inlet 148, any portions of the channel 152 defined thereby, and the sink 156. The increased rigidity of the first part 144 may also make attaching the port 130 to the first part 144 easier since pressure may be applied to the first part 144 during the attachment process substantially without deforming the first part 144.

The first part 144 exhibits a shape that is different than the second part 146. The first part 144 exhibits a first elongated shape that extends in a direction that is generally perpendicular to the longitudinal axis 140 and the second part 146 may exhibit a second elongated shape (*e.g*., generally cylindrical shape) that extends generally parallel to the longitudinal axis 140. The first elongated shape of the first part 144 may strengthen the attachment between the first part 144 and the fluid impermeable barrier 106 by increasing the surface area of the first part 144 that is attached to the fluid impermeable barrier 106. The first elongated shape of the first part 144 also prevents or at least inhibits twisting of the sheath 102. Examples of the first elongated shape include a generally rectangular cross-sectional shape, a generally oval cross-sectional shape, a generally elliptical cross-sectional shape, or a generally diamond cross-sectional shape. In a particular example, the first elongated shape is a generally diamond cross-sectional shape since the relatively sharp corners of the generally diamond cross-sectional shape that are furthest spaced from the second part 146 facilitate attaching the fluid impermeable barrier 106 to the first part 144 without forming a gap between the first part 144, the first panel 108, and the second panel 110 compared to a more rounded corner. In a particular example, the first elongated shape included rounded corners since the rounded corners are less likely to uncomfortably press into the individual than sharp corners. The second elongated shape of the second part 146 that extends parallel to the longitudinal axis 140 may facilitate attachment to the conduit 142.

The first part 144 exhibits a first maximum thickness and the second part 146 exhibits a second maximum thickness. As discussed in more detail elsewhere, the sheath 102 is configured to lie substantially flat when disposed on a flat surface which allows the fluid collection assembly 100 to be worn discreetly, prevents or at least inhibits pooling of bodily fluids in the chamber 112, and allows the fluid collection assembly 100 to be used with buried penises. It is noted that "substantially flat" allows for deviations that generally do not prevent the fluid collection assembly 100 from being worn discretely, still inhibits pooling of bodily fluids, and allows the fluid collection assembly 100 to be used by a buried penis, such as deviations caused by the port 130, the vents 134, or minor bulges or waves formed in the fluid impermeable barrier 106. Generally, the port 130 is configured to minimize the first maximum thickness and the second maximum thickness thereby preventing or at least inhibiting the ability of the sheath 102 to lay flat. The second maximum thickness is generally dictated by the need to attach the conduit 142 to the second part 146. For example, the second maximum thickness may need to be slightly greater than the inner diameter of the conduit 142 when the second part 146 is a male connector and greater than the outer diameter of the conduit 142 when the second part 146 is a female connector. In an particular example, the second part 146 is a male connector since the second maximum thickness of the second part 146 is smaller when the second part 146 is a male connector than when the second part 146 is a female connector. It is noted that, when the second part 146 is a male connector, the second part 146 has less of an adverse effect on the ability of the sheath 102 to lay flat than if the conduit 142 is directly attached to the fluid impermeable barrier 106 since the second maximum thickness may be less than an outer diameter of the conduit 142.

Generally, the first maximum thickness of the first part 144 is substantially equal to or less than the second maximum thickness to minimize the adverse effect that the first part 144 has on the ability of the sheath 102 to lay flat. In an example, when the second maximum thickness is at or near an intersection between the first and second parts 144, 146, the first maximum thickness may be substantially equal to the second maximum thickness to prevent formation of corners that may press uncomfortably into the individual. The second maximum thickness may be at or near the intersection between the first and second parts 144, 146 when the outer surface 154 of the second part 146 is tapered.

In an embodiment, the first maximum thickness of the first part 144 is greater than a collective thickness of the fluid impermeable barrier 106 (*e.g.*, the collective thickness of the first and second panels 108, 110) and the porous material 122. The increased thickness of the first maximum thickness of the first part 144 relative to the collective thickness of the fluid impermeable barrier 106 and the porous material 122 prevents the sheath 102 from lying flat at or near the port 130. However, it is noted that the high flexibility of the fluid impermeable barrier 106, especially when the fluid impermeable barrier 106 is formed from polyurethane, minimizes the percentage of the sheath 102 adjacent to the port 130 that does not lie flat. Further, the first part 144 is at least one of spaced from the opening 114 which prevents the first maximum thickness from affecting the ability of the fluid collection assembly 100 to be used with a buried penis, is located at the greatest air flow which inhibits pooling of bodily fluids, or the first maximum thickness is less than the maximum thickness of the conduit 142 thereby causing the conduit 142 to more adversely affect the ability to use the fluid collection assembly 100 discretely than the port 130. In an example, the first maximum thickness may be greater than the collective thickness of the fluid impermeable barrier 106 and the porous material 122 by about 500% (*i.e.*, 5 times greater) or less, about 400% or less, about 300% or less, about 200% or less, or about 150% or less. It is noted that the first maximum thickness may be larger than the collective thickness of the fluid impermeable barrier 106 and the porous material 122 to allow the inlet 148 to be sufficiently large to remove the bodily fluids from the chamber 112 and may be dictated by the size of the conduit 142.

The first part 144 of the port 130 may define a sink 156 that is distinct from the inlet 148. The sink 156 is positioned upstream from the inlet 148. For example, the inlet 148 may exhibit dimension(s) (*e.g.*, width and/or thickness) that is comparable to corresponding dimension(s) of the channel 152 are or near the intersection of the first part 144 and the second part 146. The dimension(s) of the inlet 148 are comparable to the corresponding dimension(s) of the channel 152 when the dimension(s) of the inlet 148 differ from the dimension(s) of the channel 152 by at most about ±10% or at most about ±5%. For example, as illustrated, the inlet 148 exhibits a width that is substantially the same as the width of the channel 152 at the intersection of the first and second parts 144, 146. The sink 156 exhibits a maximum dimension that is greater than a corresponding dimension(s) of the inlet 148 and is not comparable to a corresponding dimension(s) of the channel 152 at the intersection of the first and second parts 144, 146. For example, as illustrated, the sink 156 defined by the first part 144 exhibits a maximum dimension (*i.e.*, width) that is significantly greater than the corresponding width of the inlet 148 and the corresponding width of the channel 152 at the intersection of the first and second parts 144, 146. It is noted that the wider width of the first part 144 relative to the second part 146 allows the first part 144 to define the sink 156.

The sink 156 facilitates flow of the bodily fluids through the chamber 112, into the port 130, and through the port 130. For example, the wider dimension(s) of the sink 156 relative to the inlet 148 makes it easier for the bodily fluids to enter the port 130 than a substantially similar port 130 that does not include the sink 156 (*i.e.*, the inlet 148 is the inlet of the port 130). The wider width of the sink 156 relative to the inlet 148 also distributes the air flow to a greater percentage of the chamber 112 than a substantially similar port 130 that does not include the sink 156. The wider dimension(s) of the sink 156 relative to the inlet 148 increases the unoccupied volume of the port 130 than if the port 130 did not include the sink 156. The greater volume of the port 130 allows the port 130 to receive and hold a greater volume of bodily fluids thereby inhibiting oversaturation of the porous material 122 with the bodily fluids since the port 130 may temporarily store the bodily fluids in addition to the porous material 122. In other words, the sink 156 may form a fluid reservoir. Also, it has been found that the sink 156 decreases turbulent flow of the bodily fluids flowing through the port 130 than if the port 130 did not include the sink 156. The decreased turbulent flow allows the bodily fluids to flow through the port 130 at a greater rate than if the port 130 did not include the sink 156 thereby inhibiting oversaturation of the bodily fluids in the porous material 122. The decreased turbulent flow of the bodily fluids caused by the sink 156 may especially facilitate the operation of the fluid collection assembly 100 when the second port 146 forms a male connector. For example, forming the second part 146 as a male connector decreases the lateral dimension(s) (*e.g*., diameter) of the channel 152 which causes the lateral dimensions of the second part 146 to limit the rate at which bodily fluids are removed from the chamber 112. The decreased turbulent flow of the bodily fluids through the port 130 caused by the sink 156 helps mitigate this issue.

In an embodiment, the dimension(s) of the sink 156 are maintained substantially constant. Maintaining the dimension(s) of the sink 156 substantially constant maximizes the volume of the sink 156, and by extension the volume of the port 130, that may be a fluid reservoir. However, maintaining the dimension(s) of the sink 156 substantially constant may cause a step to form at the intersection of the inlet 148 and the sink 156 which may increase turbulent flow of the bodily fluids flowing through the port 130 than if there was not step between the inlet 148 and the sink 156. In an embodiment, as illustrated, the dimension(s) of the sink 156 may be tapered along at least a portion of the length (*e.g*., measured parallel to the longitudinal axis 140) of the sink 156. In other words, the dimension(s) of the sink 156 may vary along at least a portion of the length thereof. The tapered dimension(s) of the sink 156 may prevent or at least decrease the step at the intersection of the inlet 148 and the sink 156. Thus, the tapered dimension(s) may decrease turbulent flow of the bodily fluids flowing through the port 130 than if the dimension(s) of the sink 156 where maintained substantially constant. It is noted that the tapered dimension(s) of the sink 156 decreases the volume of bodily fluids that may be temporarily stored in the sink 156 and, by extension, the port 130.

Due to the flexibility of the fluid impermeable barrier 106, the sheath 102 may bend to exhibit a generally L- or U-shape which may cause the fluid impermeable barrier 106 to extend across the inlet of the port 130 (*e.g*., extend across the sink 156 or the inlet 148 when the port 130 does not include the sink 156). For example, during use, the fluid collection assembly 100 may be positioned on the individual such that the fluid collection assembly 100 generally extends from the pubic region of the individual towards the feet of the individual. However, in many hospitals, the vacuum source is positioned behind the bed of the individual such that the conduit 142 extends from the fluid outlet 118 towards the head of the individual when the individual is lying on the bed. As such, the conduit 142 may cause the sheath 102 to bend and exhibit the generally L- or U-shape. It has been found that the fluid impermeable barrier 106 may obstruct the inlet of the port 130 thereby preventing or inhibiting bodily fluids entering the port 130 when the sheath 102 bends and exhibits the generally L- or U-shape. As such, in some embodiments, the port 130 may include at least one tab 158 extending from the first part 144 into the chamber 112.

The tab 158 extending from the first part 144 increases the likelihood that a passageway is formed that allows bodily fluids to flow to the port 130 even when the sheath 102 bends and exhibits the generally L- or U-shape. In other words, the tab 158 may allow bodily fluids to continue to flow into the port 130 and/or increase the rate at which the bodily fluids may flow into the port 130 when the sheath 102 is bent.

The tab 158 may extend from the first part 144 by a distance that is about 2 mm or more, about 3 mm or more, about 4 mm or more, about 5 mm or more, about 6 mm or more, about 7 mm or more, about 8 mm or more, about 9 mm or more, about 1 cm or more, about 1.25 cm or more, about 1.5 cm or more, or in ranges of about 1 mm to about 3 mm, about 2 mm to about 4 mm, about 3 mm to about 5 mm, about 4 mm to about 6 mm, about 5 mm to about 7 mm, about 6 mm to about 8 mm, about 7 mm to about 9 mm, about 8 mm to about 1 cm, about 9 mm to about 1.25 cm, or about 1 cm to about 1.5 cm. Generally, the ability of the tab 158 to allow bodily fluids to continue to flow into the port 130 and/or increase the rate at which the bodily fluids may flow into the port 130 when the sheath 102 is bent is improved as the distance that the tab 158 extends from the first part 144 is increased. However, increasing the distance that the tab 158 extends from the first part 144 increases the likelihood that the tab 158 uncomfortably presses into the individual, for example, when the sheath 102 is bent. The likelihood that the tab 158 uncomfortably presses into the individual may be mitigated by cushioning the tab 158 with the porous material 122. The tab 158 may be cushioned by the porous material 122 when the tab 158 is formed on a side of the first part 144 adjacent to the first panel 108 and configuring the porous material 122 to be positioned between the tab 158 and the second panel 110.

In an embodiment, the tab 158 may be formed from the same material as the rest of the port 130. In such an embodiment, the port 130 may exhibit integral construction (*e.g*., single-piece construction) which may make manufacturing of the port 130 more efficient. In an embodiment, the tab 158 may be distinct (*e.g*., formed from a different material) from the rest of the port 130. In such an embodiment, manufacturing of the port 130 may be more difficult since manufacturing of the port 130 requires forming to pieces (instead of one) and attaching the two pieces together.

Referring back to **FIGS. 1A-1E**, the fluid outlet 118 and the port 130 may be located at or near the distal region 162 of the sheath 102 which is expected to be the gravimetrically low point of the chamber 112 when worn by a user. Locating the fluid outlet 118 and the port 130 at or near the distal region 162 of the sheath 102 enables the conduit 142 to receive more of the bodily fluids than if the fluid outlet 118 and the port 130 was located elsewhere and reduce the likelihood of pooling (e.g., pooling of the bodily fluids may cause microbe growth and foul odors). For instance, the bodily fluids in porous material 122 due to capillary forces. However, the bodily fluids may exhibit a preference to flow in the direction of gravity, especially when at least a portion of the porous material 122 is saturated with the bodily fluids. Accordingly, the fluid outlet 118 and the port 130 may be located in the fluid collection assembly 100 in a position expected to be the gravimetrically low point in the fluid collection assembly 100 when worn by a user.

As previously discussed, the sheath 102 includes at least one porous material 122 disposed in the chamber 112. The porous material 122 may direct the bodily fluids to one or more selected regions of the chamber 112, such as away from the penis and towards the fluid outlet 118. As such, the porous material 122 may facilitate the removal of the bodily fluids from the chamber 112 and form a padding layer that prevents the penis from resting against a damp material which may cause degradation of the skin of the penis and/or make the fluid collection assembly 100 more uncomfortable to wear. The porous material 122 may also blunt a stream of urine from the penis.

In an embodiment, the porous material 122 includes a wicking material configured to wick any bodily fluids away from the opening 114 thereby preventing the bodily fluids from escaping the chamber 112. Such "wicking" may not include absorption of fluid into the wicking material. Put another way, substantially no absorption of fluid into the material may take place after the material is exposed to the fluid and removed from the fluid for a time. While no absorption is desired, the term "substantially no absorption" may allow for nominal amounts of absorption of fluid into the wicking material (*e.g*., absorbency), such as less than about 30 wt% of the dry weight of the wicking material, less than 20 wt%, less than 15 wt%, less than 10 wt%, less than about 7 wt%, less than about 5 wt%, less than about 3 wt%, less than about 2 wt%, less than about 1 wt%, or less than about 0.5 wt% of the dry weight of the wicking material. The wicking material may also wick the fluid generally towards an interior of the chamber 112, as discussed in more detail below. In an embodiment, the porous material 122 is configured to adsorb or absorb the bodily fluids. Similar, to the wicking material, such adsorbing or absorbing material may move bodily fluids away from the opening 114 thereby preventing the bodily fluids from escaping the chamber 112.

The porous material 122 may be formed from any suitable porous material. For example, the porous material 122 may be formed from nylon (*e.g*., spun nylon fibers), polyester, polyurethane, polyethylene, polypropylene, other porous polymers, hydrophobic foam, an open cell foam, wool, silk, linen, cotton (*e.g*., cotton gauze), felt, other fabrics, a coated porous material (*e.g*., a water repellent coated porous material), any other suitable porous materials, or combinations thereof.

**FIG. 1G** is a cross-sectional schematic of a porous material 122, according to an embodiment. The porous material 122 includes a first layer 164 and a second layer 166. The first and second layers 164, 166 may be a woven material. The porous material 122 also includes a plurality of fibers 168 forming a layer between the first layer 164 and the second layer 166. Each of the first layer 164, the second layer 166, and the plurality of fibers 168 define a plurality of pores, thereby allowing transport of the bodily fluids and air circulation through the porous material 122. The pores defined by the plurality of fibers 168 may be at least one of larger or more numerous, thereby decreasing the likelihood that dried bodily fluids clog the porous material 122. The presence of the plurality of fibers 168 also cause the porous material 122 feel soft against the penis and provides a cushioning effect to the penis. The plurality of fibers 168 may also prevent the vacuum force from collapsing the porous material.

In an embodiment, the plurality of fibers may cause the first layer 164 to be spaced from the second layer 166 by a distance d. The distance d may be selected based on the number of fibers that form the plurality of fibers 168 and the density at which the plurality of fibers 168 are packed together. For example, the distance d may be selected to be about 0.25 mm or more, about 0.5 mm or more, about 0.75 mm or more, about 1 mm or more, about 1.5 mm or more, about 2 mm or more, about 3 mm or more, about 4 mm or more, about 5 mm or more, about 6 mm or more, about 8 mm or more, about 10 mm or more, about 12.5 mm or more, about 15 mm or more, or in ranges of about 0.25 mm to about 0.75 mm, about 0.5 mm to about 1 mm, about 0.75 mm to about 1.5 mm, about 1 mm to about 2 mm, about 1.5 mm to about 3 mm, about 2 mm to about 4 mm, about 3 mm to about 5 mm, about 4 mm to about 6 mm, about 5 mm to about 8 mm, about 6 mm to about 10 mm, about 8 mm to about 12.5 mm, or about 10 mm to about 15 mm. The thickness of the distance d may be selected to adjust the absorbency of the porous material 122. For example, increasing the thickness may increase the volume of the plurality of fibers 168 and/or the porosity defined by the plurality of fibers 168 which increases the amount of bodily fluids that may be received by and at least partially stored in the porous material 122.

The first layer 164, the second layer 166, and the plurality of fibers 168 may be formed from any suitable material, such as a hydrophobic material, a hydrophilic material, polyester, cotton, or any other porous material disclosed herein. In an embodiment, one or more of the first layer 164, the second layer 166, or the plurality of fibers 168 are formed from a hydrophobic material that inhibits the porous material 122 from storing the bodily fluids therein which may facilitate removal of the bodily fluids from the chamber 112. In an embodiment, one or more of the first layer 164, and second layer 166, or the plurality of fibers 168 are formed from a hydrophilic material which allows the porous material 122 to temporarily store the bodily fluids therein thereby limiting the quantity of bodily fluids that pool around the skin of the individual. In an embodiment, two or more of the first layer 164, the second layer 166, or the plurality of fibers 168 are formed from different materials. In such an embodiment, the first layer 164 may define the penis receiving area 131 or is otherwise closer to the penis receiving area 131 than the second layer 166. The first layer 164 may be formed from a hydrophobic material while the plurality of fibers 168 are formed from a hydrophilic material. Such a configuration may cause the bodily fluids to be pulled through the first layer 164 and temporarily stored in the plurality of fibers 168. However, the first layer 164 may remain substantially dry due to the hydrophobicity thereof which allows the porous material 122 to feel dry to the penis. In an embodiment, at least one of the first layer 164 or the second layer 166 is formed from a fabric (*e.g*., fabric gauze) and the plurality of fibers 168 is formed from nylon fibers (*e.g*., spun nylon fibers).

In an embodiment, not shown, the porous material 122 may be formed from two layers instead of the three layers illustrated in **FIG. 1G****.** For example, the porous material 122 may be formed from a fluid permeable membrane and a fluid permeable support. The fluid permeable support may define or otherwise be closer to the penis receiving area 131 than the fluid permeable support. The fluid permeable membrane may be composed and/or structured to wick bodily fluids away from the penis receiving area 131, thereby minimizing the quantity of bodily fluids that are present in the penis receiving area 131 or otherwise present against the skin of the individual. It is also noted that the fluid permeable membrane may also be configured to adsorb or absorb the bodily fluids to minimize the quantity of bodily fluids that are present in the penis receiving area 131 or otherwise present against the skin of the individual. The fluid permeable membrane may be formed from any of the porous materials disclosed herein. For example, the fluid permeable membrane may be formed from fabric, such as a gauze (*e.g*., silk, linen, or cotton gauze), another soft fabric, another smooth fabric, a woven or nonwoven material (*e.g*., spun nylon fiber), or any other suitable porous material. Forming the fluid permeable membrane from gauze, soft fabric, and/or smooth fabric (or any of the other porous materials 122 disclosed herein that may contact the penis) may reduce chaffing caused by the fluid collection assembly 100.

The fluid permeable support is configured to support the fluid permeable membrane since the fluid permeable membrane may be formed from a relatively foldable, flimsy, or otherwise easily deformable material. For example, the fluid permeable support may be positioned such that the fluid permeable membrane is disposed between the fluid permeable support and the fluid impermeable barrier 106. As such, the fluid permeable support may support and maintain the position of the fluid permeable membrane. The fluid permeable support may include any of the fluid permeable membrane materials disclosed herein above. For example, the fluid permeable membrane material(s) may be utilized in a more dense or rigid form than in the fluid permeable membrane when used as the fluid permeable support. The fluid permeable support may be formed from any fluid permeable material that is less deformable than the fluid permeable membrane. For example, the fluid permeable support may include a porous polymer (*e.g*., nylon, polyester, polyurethane, polyethylene, polypropylene, etc.) structure or an open cell foam. In some examples, the fluid permeable support may be formed from a natural material, such as cotton, wool, silk, or combinations thereof. In such examples, the material may have a coating to prevent or limit absorption of fluid into the material, such as a water repellent coating. In some examples, the fluid permeable support may be formed from fabric, felt, gauze, or combinations thereof.

In an embodiment, the porous material 122 may include a single layer (*e.g*., one of the first layer 164, the second layer 166, the layer formed from the plurality of fibers 168, the fluid permeable membrane, the fluid permeable support, or another porous layer). In an embodiment, the porous material 122 may be formed from four or more layers.

Examples of other materials that may form the porous material 122 are disclosed in U.S. Provisional Patent Application No. 63/134,754 filed on January 7, 2021; U.S. Provisional Patent Application No. 63/241,575 filed on September 8, 2021; and U.S. Provisional Patent Application No. 63/247,491 filed on September 23, 2021.

In an embodiment, the porous material 122 may be a sheet (*e.g*., a multi-layer sheet). The porous material 122 is a sheet when the porous material 122 at least one of is generally planar when lying on a flat surface, does not define a cavity (*e.g*., is not tubular), or exhibits a length and width that is greater than a thickness thereof. Forming the porous material 122 as a sheet may facilitate the manufacturing of the fluid collection assembly 100. For example, forming the porous material 122 as a sheet allows the first panel 108, the second panel 110, and the porous material 122 to each be sheets. During the manufacturing of the fluid collection assembly 100, the first panel 108, the second panel 110, and the porous material 122 may be stacked and then attached to each other in the same manufacturing step. For instance, the porous material 122 may exhibit a shape that is the same size or, more preferably, slightly smaller than the size of the first panel 108 and the second panel 110. As such, attaching the first panel 108 and the second panel 110 together along the outer edges (*e.g*., top edges 136 and lateral edges 138) thereof may also attach the porous material 122 to the first panel 108 and the second panel 110. The porous material 122 may be slightly smaller than the first panel 108 and the second panel 110 such that the first panel 108 and/or the second panel 110 extend around the porous material 122 such that the porous material 122 does not form a passageway through the fluid impermeable barrier 106 through which the bodily fluids may leak. Also, attaching the porous material 122 to the first panel 108 and/or the second panel 110 may prevent the porous material 122 from significantly moving in the chamber 112, such as preventing the porous material 122 from bunching together near the fluid outlet 118. In an example, the porous material 122 may be attached to the first panel 108 or the second panel 110 (*e.g*., via an adhesive) before or after attaching the first panel 108 to the second panel 110. In an example, the porous material 122 may merely be disposed in the chamber 112 without attaching the porous material 122 to at least one of the first panel 108 or the second panel 110. In an embodiment, as will be discussed in more detail below, the porous material 122 may exhibit shapes other than a sheet, such as a hollow generally cylindrical shape.

The porous material 122 may exhibit a shape that generally corresponds to the shape of the fluid impermeable barrier 106 such that the porous material 122 partially or substantially completely occupies all of the chamber 112. For example, as illustrated, the porous material 122 may exhibit a generally bullet shape. The porous material 122 may exhibit a generally bullet shape when the porous material 122 includes a proximal portion 170 corresponding to the proximal region 160 and a distal portion 172 extending from the proximal portion 170 that corresponds to the distal region 162. The proximal portion 170 may exhibit a substantially constant first width and the distal portion 172 may exhibit a second width that is less than the first width. The second width may be constant or vary (*e.g*., vary at a constant or variable rate). The porous material 122 may exhibit a shape other than the generally bullet shape, such as a generally rectangular shape, generally semi-oval shape. or any other suitable shape.

Generally, the sheath 102 is substantially flat when the penis is not in the penis receiving area 131 and the sheath 102 is resting on a flat surface. The sheath 102 is substantially flat because the fluid impermeable barrier 106 is formed from the first panel 108 and the second panel 110 instead of a generally tubular fluid impermeable barrier. Further, as previously discussed, the porous material 122 may be a sheet, which also causes the sheath 102 to be substantially flat. It is noted that the sheath 102 is described as being substantially flat because at least one of the porous material 122 may cause a slight bulge to form in the sheath 102 depending on the thickness of the porous material 122, the port 130 may cause a bulge thereabout, or the base 104 may pull on portions of the sheath 102 thereabout. It is also noted that the sheath 102 may also be compliant and, as such, the sheath 102 may not be substantially flat during use since, during use, the sheath 102 may rest on a non-flat surface (*e.g*., may rest on the testicles, the perineum, and/or between the thighs) and the sheath 102 may conform to the surface of these shapes.

The ability of the sheath 102 to be substantially flat when the penis is not in the penis receiving area 131 and the sheath 102 is resting on a flat surface allows the fluid collection assembly 100 to be used with a buried and a non-buried penis. For example, when the fluid collection assembly 100 is being used with a buried penis, the penis does not extend into the penis receiving area 131 which causes the sheath 102 to lie relatively flat across the aperature 124. When the sheath 102 lies relatively flat across the aperture 124, the porous material 122 extends across the aperature and is in close proximity to the buried penis. As such, the porous material 122 prevents or inhibits pooling of bodily fluids discharged from the buried penis against the skin of the individual since the porous material 122 will receive and remove at least a significant portion of the bodily fluids that would otherwise pool against the skin of the individual. Thus, the skin of the individual remains dry thereby improving comfort of using the fluid collection assembly 100 and preventing skin degradation. However, unlike other conventional fluid collection assemblies that are configured to be used with buried penises, the fluid collection assembly 100 may still be used with a non-buried penis since the non-buried penis can still be received into the penis receiving area 131, even when the penis is fully erect. Additionally, the ability of the sheath 102 to be substantially flat allows the fluid collection assembly 100 to be used more discretely than if the sheath 102 was not substantially flat thereby avoiding possibly embarrassing scenarios.

When the sheath 102 is substantially flat, the porous material 122 may occupy substantially all of the chamber 112 and the penis receiving area 131 is collapsed (shown as being non-collapsed in **FIGS. 1C** and **1D** for illustrative purposes). In other words, the sheath 102 may not define a region that is constantly unoccupied by the porous material 122. When the porous material 122 occupies substantially all of the chamber 112, the bodily fluids discharged into the chamber 112 are unlikely to pool for significant periods of time since pooling of the bodily fluids may cause sanitation issues, cause an odor, and/or may cause the skin of the individual to remain in contact with the bodily fluids which may cause discomfort and skin degradation.

As previously discussed, the first panel 108, the second panel 110, and the porous material 122 may be selected to be relatively flexible. The first panel 108, the second panel 110, and the porous material 122 are relatively flexible when the first panel 108, the second panel 110, and the porous material 122, respectively, are unable to maintain their shape when unsupported. The flexibility of the first panel 108, the second panel 110, and the porous material 122 may allow the sheath 102 to be substantially flat, as discussed above. The flexibility of the first panel 108, the second panel 110, and the porous material 122 may also allow the sheath 102 to conform to the shape of the penis even when the size and shape of the penis changes (*e.g*., becomes erect) and to minimize any unoccupied spaces in the chamber 112 in which bodily fluids may pool.

As previously discussed, the fluid collection assembly 100 includes a base 104 that is configured to be coupled to the skin that surrounds the penis (*e.g*., mons pubis, thighs, testicles, and/or perineum) and have the penis disposed therethrough. For example, the base 104 may define an aperture 124 configured to have the penis positioned therethrough. The base 104 may be flexible, thereby allowing the base 104 to conform to any shape of the skin surface and mitigate the base 104 pulling the on skin surface.

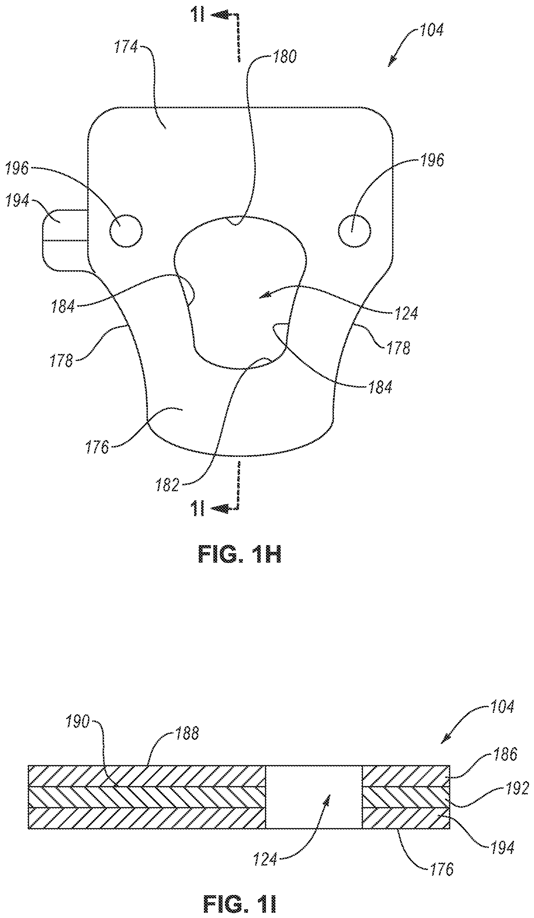

**FIG. 1H** is a top plan view of the base 104, according to an embodiment. The base 104 is configured to be attached to the region about the penis (*e.g*., mons pubis) while not being attached to the thighs since attaching the base 104 to the thighs may cause the base 104 to uncomfortably pull when the individual moves. In an example, the base 104 may include a primary attachment portion 174 and a secondary attachment portion 176. The primary attachment portion 174 is configured to be attached to the mons pubis and the secondary attachment portion 176 is configured to be attached to the region about the penis except for the mons pubis (*e.g*., an upper portion of the testicles). The primary attachment portion 174 may exhibit a generally rectangular or trapezoidal shape. The primary attachment portion 174 may extend further from the aperture 124 than the secondary attachment portion 176 since the mons pubis is less sensitive and larger than the other regions about the penis. The secondary attachment portion 176 may include one or more concave lateral edges 178. The concave lateral edges 178 may help prevent the secondary attachment portion 176 from being attached to the thighs of the individual. It is noted that any corners of the base 104 may be rounded to prevent or at least inhibit the base 104 uncomfortably pressing into the individual.

The base 104 may exhibit shapes other than the shape illustrated in **FIG. 1H****.** For example, the base 104 may exhibit a generally partially triangular shape having three apexes and edges extending between each of the apexes. The apexes may be rounded to prevent the base 104 from digging into and hurting the individual. The aperture 124 may be located off-center and closer to one of the apexes than the other apexes. Other examples of shapes that the base 104 may form are disclosed in PCT Application No. PCT/US2021/015787 filed on January 29, 2021.

In an embodiment, not shown, the aperture 124 may exhibit a generally circular shape. In an embodiment, as illustrated, the aperture 124 may exhibit a non-circular shape. The non-circular shape of the aperture 124 may be selected to at least one of correspond to the cross-sectional shape of a base of a penis and/or better conform to the region about the penis, either of which may limit leakage and pooling of the bodily fluids. For example, as illustrated, the aperture 124 may exhibit a generally bell-like shape. The generally bell-like shape includes a concave (relative to an interior of the aperture 124) top edge 180 and a concave bottom edge 182 opposite the concave top edge 180. The generally bell-like shape also includes two convex lateral edges 184 extending between the top and bottom edges 180, 182. Any corners between the top, bottom, and lateral edges 180, 182, 184 may be rounded. The two convex lateral edges 184 cause the width of the aperture 124 to vary. For example, the two convex lateral edges 184 cause a width of the aperture 124 near the top edge 180 to be smaller than a width of the aperture 124 near the bottom edge 182. The generally bell-like shape of the aperture 124 may allow the aperture 124 to correspond to the shape of the base of the penis which prevents bodily fluids leaking from the chamber 112 and inhibits pooling of the bodily fluids. The aperture 124 may exhibit other non-circular shapes, such as generally triangular shape, a generally trapezoidal shape, a generally hippopede shape, or any other suitable non-circular shape.

**FIG. 1I** is a cross-sectional schematic of the base 104, according to an embodiment. The base 104 includes a substrate 186 having a top surface 188 and a bottom surface 190. The top surface 188 is closer to the sheath 102 than the bottom surface 190 while the bottom surface 190 is closer to the skin of the individual than the top surface 188. The base 104 may also include an adhesive layer 192 disposed on at least a portion of the bottom surface 190. The adhesive layer 192 is configured to attach the base 104 to the skin around the penis. The base 104 may also include a release liner 194 is configured to be easily removed from the adhesive layer 192 and is configured to prevent the adhesive layer 192 inadvertently becoming attached to an object.

The substrate 186 may be formed from a fluid impermeable material to prevent bodily fluids from leaking from the chamber 112 through the base 104. For example, the substrate 186 may be formed from any of the fluid impermeable materials disclosed herein. In a particular example, the substrate 186 may be formed from a hydrophobic porous material, such as a hydrophobic woven or nonwoven material. The hydrophobicity of the hydrophobic porous material, in conjunction with the small pores thereof, may prevent bodily fluids from flowing therethrough. It is noted that the hydrophobic porous material may allow air to flow therethrough which may make wearing the fluid collection assembly 100 more comfortable.

In an embodiment, the substrate 186 exhibits a thickness that is about 2 mm or less, about 1.5 mm or less, about 1 mm or less, about 0.75 mm or less, about 0.5 mm or less, about 0.3 mm or less, about 0.2 mm or less, about 0.1 mm or less, about 0.05 mm or less, or in ranges of about 0.05 mm to about 0.2 mm, about 0.1 mm to about 0.3 mm, about 0.2 mm to about 0.5 mm, about 0.3 mm to about 0.75 mm, about 0.5 mm to about 1 mm, about 0.75 mm to about 1.5 mm, or about 1 to about 2 mm. Forming the substrate 186 to exhibit any of the above thicknesses may cause the substrate 186 to be sufficiently flexible to conform to the shape and contours of the skin surrounding the penis. For instance, the shape and contours of the skin surrounding the penis may vary from individual to individual and configuring the substrate 186 to be a thin film may allow the base 104 to conform to the shape and contour of the skin surrounding the penis while preventing the formation of any gaps between the base 104 and the skin through which bodily fluids may leak. Further, the thin film substrate 186 is able to be attached to the skin of the individual without or substantially without pulling the skin of the individual which makes the fluid collection assembly 100 more comfortable to use for prolonged periods of time. It is noted that the amount of the fluid collection assembly 100 pulls on the skin decreases with decreasing thickness of the substrate such that, for example, a substrate exhibiting a thickness of about 2 mm pulls more on the skin than a substantially similar substrate exhibiting a thickness of about 0.5 mm.

The adhesive layer 192 may be formed from any adhesive that may safely attach the substrate 186 to the skin surrounding the penis. In an example, the adhesive layer 192 may be formed from a silicone-based adhesive, such as a silicone-gel adhesive. Silicone-based adhesives, such as Medical Silicone Tape 2475P available from 3M, has been found to secure the fluid collection assembly 100 to the skin surrounding the penis for at least 24 hours, even immediately after cleaning the skin surface with a wipe. In an example, the adhesive layer 192 may be formed from an acrylic gel adhesive or a hydrogel.

In an embodiment, the base 104 is at least partially transparent (e.g., the substrate 186 and the adhesive layer 192 are formed from at least partially transparent materials). In such an embodiment, a person (*e.g*., medical practitioner) may be able to examiner the skin surrounding the penis, such as to determine the health of the skin. Further, the person may be able to detect any gaps between the base 104 and the skin of the individual through which bodily fluids may leak. A person may be able to eliminate the gaps or replace the fluid collection assembly 100 after detecting the gaps to prevent leaks and prevent degradation of the skin caused by the skin being in contact with the bodily fluids.

As previously discussed, the base 104 is configured to be attached (*e.g*., permanently attached) to the sheath 102. The base 104 is configured to be attached to the sheath 102 when, for example, the fluid collection assembly 100 is provided with the base 104 attached to the sheath 102 or the base 104 is provided without being attached to the sheath 102 but is configured to be attached to the sheath 102 at some point in the future. The base 104 may be attached to the sheath 102 using any suitable technique. For example, the base 104 may be attached to the sheath 102 using an adhesive, sewing, heat sealing, impulse heating, RF welding, or US welding.

In an embodiment, the base 104 is permanently attached to the sheath 102. The base 104 is permanently attached to the sheath 102 when the base 104 cannot be detached from the sheath 102 without damaging at least one of the sheath 102 or the base 104, using a blade to separate the sheath 102 from the base 104, using chemicals to dissolve the adhesive that attaches the sheath 102 to the base 104, and/or using heat to melt or soften the adhesive or attachment (*e.g*., ultrasonic weld) that attached the sheath 102 to the base 104. In an embodiment, the base 104 is attached to the sheath 102 using an non-permanent attachment.

In an embodiment, all of the base 104 is attached to the sheath 102 which may strengthen the attachment between the sheath 102 and the base 104. In an embodiment, as illustrated, only the inner portions of the base 104 defining or adjacent to the aperture 124 are attached to the sheath 102. Attaching only the inner portions of the base 104 to the sheath 102 may be sufficient to maintain the base 104 attached to the sheath 102 during use. Only attaching the inner portions of the base 104 to the sheath 102 may allow the outer portions of the base 104 (*e.g*., portions of the base 104 other than the inner portions) to be easily handled by a user of the fluid collection assembly 100 which, in turn, makes it easier to attach the base 104 to the individual.

In an embodiment, the base 104 may be attached to the second panel 110 before the second panel 110 is attached to the first panel 108 which facilitates attaching the base 104 to sheath 102, for example, using US welding, RF welding, and impulse heating. Attaching the base 104 to the second panel 110 before securing the porous material 122 in the chamber 112 may cause difficulties securing the porous material 122 in the chamber 112 since the base 104 may be in the way. In an example, as illustrated, the base 104 and the porous material 122 may define one or more base gaps 196 and one or more porous material gaps 198, respectively. The base gaps 196 and the porous material gaps 198 may be generally aligned with each other such that the base gaps 196 and the porous material gaps 198 are adjacent to each other. The base gaps 196 and the porous material gaps 198 allows opposing portions of the fluid impermeable barrier 106 that are generally aligned with the base gaps 196 and the porous material gaps 198 to be attached together (*e.g*., using US welding, RF welding, impulse heating, direct heating, heat staking, etc.). Attaching the opposing portions of the fluid impermeable barrier 106 together may secure the porous material 122 in the chamber 112. In a particular example, as illustrated, the base gaps 196 and the porous material gaps 198 may be formed in portions of the base 104 and the porous material 122 that are adjacent to or above (*e.g*., further spaced from the fluid outlet 118) portions of the aperture 124 that are furthest spaced from the fluid outlet 118. Such positioning of the base gaps 196 and the porous material gaps 198 may keep the porous material 122 extending across the aperture 124.

Referring back to **FIGS. 1A-1D****,** as previously discussed, the fluid collection assembly 100 includes a conduit 142. The conduit 142 may include a flexible material such as plastic tubing (*e.g*., medical tubing). Such plastic tubing may include a thermoplastic elastomer, polyvinyl chloride, ethylene vinyl acetate, polytetrafluoroethylene, etc., tubing. In some examples, the conduit 142 may include silicon or latex. In some examples, the conduit 142 may include one or more portions that are resilient, such as to by having one or more of a diameter or wall thickness that allows the conduit to be flexible.

As described in more detail below, the conduit 142 is configured to be coupled to, and at least partially extend between, one or more of the fluid storage container (not shown) and the vacuum source (not shown). In an example, the conduit 142 is configured to be directly connected to the vacuum source (not shown). In such an example, the conduit 142 may extend from the fluid impermeable barrier 106 by at least one foot, at least two feet, at least three feet, or at least six feet. In another example, the conduit 142 is configured to be indirectly connected to at least one of the fluid storage container (not shown) and the vacuum source (not shown). In some examples, the conduit is secured to a wearer's skin with a catheter securement device, such as a STATLOCK^{®} catheter securement device available from C. R. Bard, Inc., including but not limited to those disclosed in U.S. Patent Nos. 6,117,163; 6,123,398; and 8,211,063.

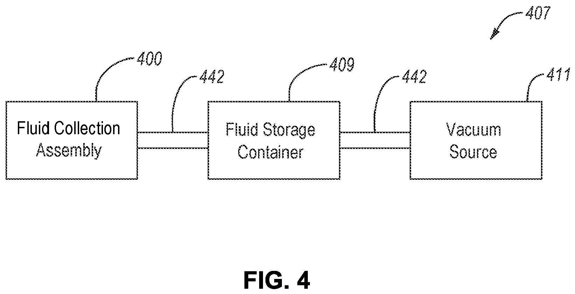

The inlet and an outlet of the conduit 142 are configured to fluidly couple (*e.g*., directly or indirectly) the vacuum source (not shown) to the chamber 112. As the vacuum source (**FIG. 4**) applies a vacuum/vacuum in the conduit 142, the bodily fluids in the chamber 112 may be drawn into the inlet 148 and out of the fluid collection assembly 100 via the conduit 142. In some examples, the conduit 142 may be frosted or opaque (*e.g*., black) to obscure visibility of the bodily fluids therein.

In some examples, the vacuum source may be remotely located from the fluid collection device. In such examples, the conduit 142 may be fluidly connected to the fluid storage container, which may be disposed between the vacuum source and the fluid collection assembly 100.

During operation, a male using the fluid collection assembly 100 may discharge bodily fluids (*e.g*., urine) into the chamber 112. The bodily fluids may pool or otherwise be collected in the chamber 112 (*e.g*., received into the porous material 122). At least some of the bodily fluids may be pulled through the interior of the conduit 142 via the inlet. The bodily fluids may be drawn out of the fluid collection assembly 100 via the vacuum/vacuum provided by the vacuum source. During operation, the vents 134 may substantially maintain the pressure in the chamber 112 at atmospheric pressure even though bodily fluids are introduced into and subsequently removed from the chamber 112.

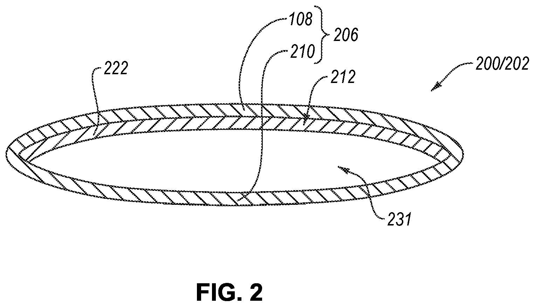

As previously discussed, the fluid collection assembly 100 is formed from the first panel 108 and the second panel 110 that are distinct sheets which may allow the first panel to be at least partially opaque while the second panel is at least partially transparent. However, forming the first and second panels 108, 110 from distinct sheets may form edges that may cause patient discomfort and requires a significant amount of manufacturing (*e.g*., welding or other attachment technique) to attach the first panel 108 to the second panel 110. As such, in some embodiments, the fluid collection assemblies disclosed herein may be formed from first and second panels that are integrally formed together (*e.g*., exhibit single piece construction) which may eliminate at least some of the edges and simplify manufacturing of such fluid collection assemblies. For example, **FIG. 2** is a cross-sectional schematic view of a fluid collection assembly 200 that includes a first panel 208 and a second panel 210 integrally formed together, according to an embodiment. Except as otherwise disclosed herein, the fluid collection assembly 200 may be the same or substantially similar to any of the fluid collection assemblies disclosed herein. For example, the fluid collection assembly 200 includes a sheath 202 and a base (not shown). The sheath 202 at least defines a chamber 212, an opening (not shown), and a fluid outlet (not shown). The fluid collection assembly 200 also includes at least one porous material 222 disposed in the chamber 212.

As previously discussed, the first panel 208 and the second panel 210 are integrally formed together. As such, the first panel 208 and the second panel 210 are different regions of fluid impermeable barrier 206 instead of different sheets that are attached together. The first panel 208 is the region of the fluid impermeable barrier 206 adjacent to the porous material 222 while the second panel 210 is the region of the fluid impermeable barrier 206 adjacent to the penis receiving area 231 (shown in a non-collapsed state for illustrative purposes).

In an embodiment, as illustrated, the first and second panels 208, 210 are formed from a thin walled tube, such as thin walled tube formed using a blown film extrusion process. Forming the first and second panels 208, 210 from the thin walled tube precludes the need to attach the longitudinal edges of the first and second panels 208, 210 together. However, the portions of the first and second panels 208, 210 that form the proximal end region and the distal end region thereof may need to be attached together using any of the techniques disclosed herein. As such, forming the first and second panels 208, 210 from a thin walled tube makes manufacturing of the fluid collection assembly 200 more efficient since fewer portions of the first and second panels 208, 210 need to be attached together. Further, forming the first and second panels 208, 210 from the thin walled tube decreases the number of edges that may press into the individual compared to forming the first and second panels 208, 210 from two distinct sheets.

In an embodiment, the first and second panels 208, 210 are formed from a single sheet that is folded. Forming the first and second panels 208, 210 from the single folded sheet precludes the need to attach one of the longitudinal edges of the first and second panels 208, 210 together. However, the portions of the first and second panels 208, 210 that form the proximal end region, the distal end region, and the portion opposite the fold may need to be attached together using any of the attachment techniques disclosed herein. Thus, forming the first and second panels 208, 210 from a single folded sheet makes manufacturing of the fluid collection assembly 200 more efficient and decreases the number of edges that are formed compared to forming the first and second panels 208, 210 from two distinct sheets. It is noted that, in either embodiment, the first panel 208 and the second panel 210 may still lie substantially flat when the penis is not in the chamber 212.

In an embodiment, the second panel 210 may have an opening formed therein. For example, the second panel 210 may have a cutout formed therein that is spaced from or extends inwardly from an outer edge of the second panel 210 (*e*.*g*., an outer edge of the thin walled tube or the single folded sheet).

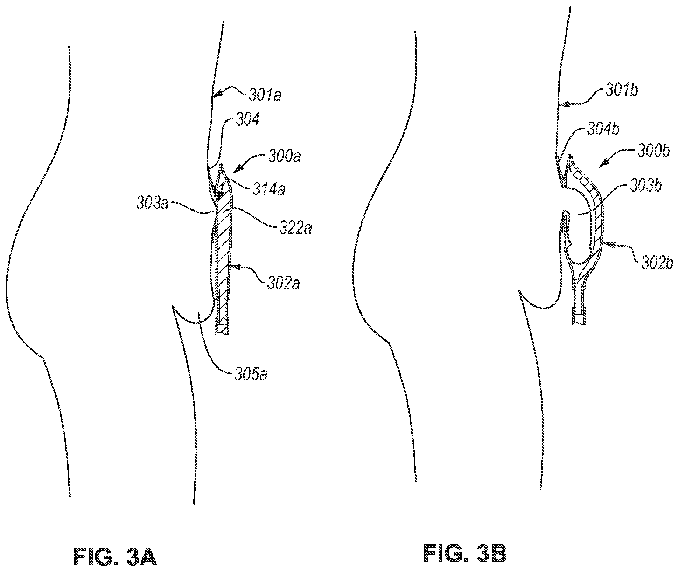

As previously discussed, the fluid collection assemblies disclosed herein may be used with a buried penis and a non-buried penis. **FIGS. 3A** and **3B** are cross-sectional schematics illustrating how substantially similar fluid collection assemblies may be used with a buried and non-buried penis, according to an embodiment. Referring to **FIG. 3A****,** a first individual 301a has a buried penis 303a (shown schematically as a slight bump). The fluid collection assembly 300a is attached to the first individual 301a. The fluid collection assembly 300a is illustrated as being substantially the same as fluid collection assembly 100 shown in **FIGS. 1A-1I****.** However, it is noted that the fluid collection assembly 300a may include any of the fluid collection assemblies disclosed herein. The fluid collection assembly 300a is attached to the first individual 301a such that the aperture of the base 304a and the opening of the sheath 302a is adjacent to the buried penis 303a. Since the fluid collection assembly 300a lies substantially flat, the porous material 322a is generally adjacent to the opening and may only be spaced from the buried penis 303a by the thickness of the base 304a which prevents almost no space for the bodily fluids to pool before being received by the porous material 322a. Is noted that the porous material 322a may be spaced from the buried penis 303a by a distance that is less than the thickness of the base 304a (*e*.*g*., the porous material 322a bulges into the aperture) or slightly greater than the thickness of the base 304a (*e.g*., the testicles 305a cause a bulge that pushes the porous material 322a away from the opening 314a or the porous material 322a exhibits a non-sheet like shape).

Referring to **FIG. 3B****,** a second individual 301b has a non-buried penis 303b. A fluid collection assembly 300b that is the same or substantially the same as the fluid collection assembly 300a is attached to the second individual 301b. The fluid collection assembly 300b is attached to the second individual 301b such that the penis 303b extends through the aperture of the base 304b and through the opening of the sheath 302b. Due to the flexibility of the fluid collection assembly 300b, the sheath 302b can receive the penis 303b therein.

**FIG. 4** is a block diagram of a system 407 for fluid collection, according to an embodiment. The system 407 includes a fluid collection assembly 400, a fluid storage container 409, and a vacuum source 411. The fluid collection assembly 400 may include any of the fluid collection assemblies disclosed herein. The fluid collection assembly 400, the fluid storage container 409, and the vacuum source 411 may be fluidly coupled to each other via one or more conduits 442. For example, fluid collection assembly 400 may be operably coupled to one or more of the fluid storage container 409 or the vacuum source 411 via the conduit 442. Bodily fluids (*e.g.*, urine or other bodily fluids) collected in the fluid collection assembly 400 may be removed from the fluid collection assembly 400 via the conduit 442 which protrudes into the fluid collection assembly 400. Vacuum force may be introduced into the chamber of the fluid collection assembly 400 via the inlet of the conduit 442 responsive to vacuum (*e.g*., vacuum) force applied at the outlet of the conduit 442.

The vacuum force may be applied to the outlet of the conduit 442 by the vacuum source 411 either directly or indirectly. The vacuum force may be applied indirectly via the fluid storage container 409. For example, the outlet of the conduit 442 may be disposed within the fluid storage container 409 and an additional conduit 442 may extend from the fluid storage container 409 to the vacuum source 411. Accordingly, the vacuum source 411 may apply vacuum to the fluid collection assembly 400 via the fluid storage container 409. The vacuum force may be applied directly via the vacuum source 411. For example, the outlet of the conduit 442 may be disposed within the vacuum source 411. An additional conduit 442 may extend from the vacuum source 411 to a point outside of the fluid collection assembly 400, such as to the fluid storage container 409. In such examples, the vacuum source 411 may be disposed between the fluid collection assembly 400 and the fluid storage container 409.

The fluid storage container 409 is sized and shaped to retain a fluid therein. The fluid storage container 409 may include a bag (*e.g*., drainage bag), a bottle or cup (*e.g.*, collection jar), or any other enclosed container for storing the bodily fluids. In some examples, the conduit 442 may extend from the fluid collection assembly 400 and attach to the fluid storage container 409 at a first point therein. An additional conduit 442 may attach to the fluid storage container 409 at a second point thereon and may extend and attach to the vacuum source 411. Accordingly, a vacuum (*e.g.*, vacuum) may be drawn through fluid collection assembly 400 via the fluid storage container 409. Fluid, such as urine, may be drained from the fluid collection assembly 400 using the vacuum source 411.

The vacuum source 411 may include one or more of a manual vacuum pump, and electric vacuum pump, a diaphragm pump, a centrifugal pump, a displacement pump, a magnetically driven pump, a peristaltic pump, or any pump configured to produce a vacuum. The vacuum source 411 may provide a vacuum or vacuum to remove fluid from the fluid collection assembly 400. In some examples, the vacuum source 411 may be powered by one or more of a power cord (*e.g*., connected to a power socket), one or more batteries, or even manual power (*e*.*g*., a hand operated vacuum pump). In some examples, the vacuum source 411 may be sized and shaped to fit outside of, on, or within the fluid collection assembly 400. For example, the vacuum source 411 may include one or more miniaturized pumps or one or more micro pumps. The vacuum sources 411 disclosed herein may include one or more of a switch, a button, a plug, a remote, or any other device suitable to activate the vacuum source 411.

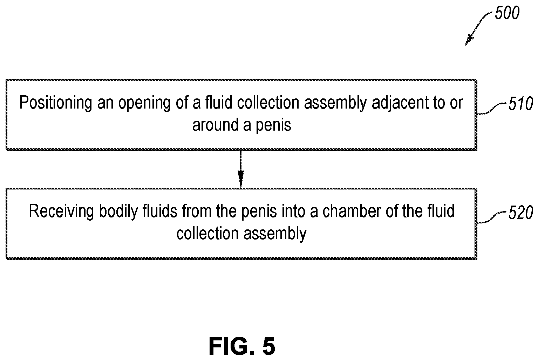

**FIG. 5** is a flow diagram of a method 500 to collect fluid, not forming part of the invention and present for background information only. The method 500 of collecting fluid may utilize use any of the fluid collection assemblies and/or fluid collection systems disclosed herein. The method 500 may include act 510, which recites "positioning an opening of a fluid collection assembly adjacent to or around a penis." Act 510 may be followed by act 520, which recites "receiving bodily fluids from the penis into a chamber of the fluid collection assembly."

Acts 510 and 520 of the method 500 are for illustrative purposes. For example, the acts 510 and 520 of the method 500 may be performed in different orders, split into multiple acts, modified, supplemented, or combined. In an example, one or more of the acts 510 or 520 of the method 500 may be omitted from the method 500.

Act 510 recites "positioning an opening of a fluid collection assembly adjacent to or around a penis." The act 510 of positioning an opening of a fluid collection assembly may include utilizing any of the fluid collection assemblies or systems disclosed herein. In some examples, act 510 may include positioning the opening such that the porous material of the fluid collection assembly extends across the opening which allows the porous material to abut or be positioned proximate to a buried penis. In some examples, act 510 may include positioned a base of a fluid collection assembly over a buried penis such that the male urethra is positioned adjacent to an aperture of the base and an opening of the sheath. In such an example, act 510 may include positioning a sheath of the male fluid collection assembly around a non-buried penis, such that at least a portion of the penis is positioned through an opening of the sheath and in the chamber of the fluid collection assembly.

Act 520 recites, "receiving bodily fluids from the penis into a chamber of the fluid collection assembly." In some examples, receiving bodily fluids from the penis into a chamber of the fluid collection assembly includes receiving the bodily fluids through the opening of the fluid collection assembly. Receiving bodily fluids from the penis into a chamber of the fluid collection assembly may include at least one of wicking, absorbing, or adsorbing the bodily fluids away from the opening using the porous material. In some examples, receiving bodily fluids from the penis into a chamber of the fluid collection assembly may include receiving the bodily fluids into the chamber of the sheath of the fluid collection assembly. Receiving bodily fluids from the penis into a chamber of the fluid collection assembly may include flowing the bodily fluids towards a portion of the chamber that is fluidly coupled to an inlet of a conduit in fluid communication a vacuum source. For instance, receiving bodily fluids from the penis into a chamber of the fluid collection assembly may include flowing the bodily fluids to a gravimetrically low point of the chamber, etc., such as via gravity, wicking, or vacuum force.

The method 500 may include applying vacuum with a vacuum source effective to vacuum the bodily fluids from the chamber via a conduit disposed or otherwise in fluid communication with the chamber. The conduit may also be fluidly coupled to the vacuum source may include using any of the vacuum sources disclosed herein. Applying vacuum with a vacuum source may include activating the vacuum source (e.g., vacuum device) in fluid communication with the inlet of the conduit in the fluid collection assembly. In some examples, activating the vacuum source in fluid communication with the inlet of the conduit in the fluid collection assembly may include supplying power to the vacuum source by one or more of flipping an on/off switch, pressing a button, plugging the vacuum source into a power outlet, putting batteries into the vacuum source, etc. In some examples, the vacuum source may include a hand operated vacuum pump and applying vacuum with a vacuum source may include manually operating the hand operated vacuum pump effective to vacuum the bodily fluids from the chamber via the conduit disposed therein that is fluidly coupled to the vacuum source.

Applying vacuum with a vacuum source effective to vacuum the bodily fluids from the chamber via a conduit disposed therein and fluidly coupled to the vacuum source may be effective to remove at least some bodily fluids from the chamber of the fluid collection assembly. In some examples, applying vacuum with a vacuum source effective to vacuum the bodily fluids from the chamber via a conduit disposed therein and fluidly coupled to the vacuum source may be effective to transfer at least some of the bodily fluids from the chamber to a fluid storage container (*e.g*., a bottle or bag).

The vacuum source (*e.g*., vacuum device) may be disposed on or within the fluid collection assembly and applying vacuum with the vacuum source may include activating the vacuum source. In some examples, the vacuum source may be spaced from the fluid collection assembly and applying vacuum with the vacuum source may include activating the vacuum source.

Applying vacuum with a vacuum source effective to vacuum the bodily fluids from the chamber via a conduit disposed therein and fluidly coupled to the vacuum source may include detecting moisture in the chamber (*e.g*., via one or more moisture sensors) and responsive thereto, activating the vacuum source to provide vacuum in the chamber. The control of the vacuum source responsive to the signals indicating that moisture or a level thereof is present in the chamber may be automatic, such as via a controller (*e.g*., computer programmed to perform the operation), or may merely provide an indication that a level of moisture is present that may necessitate removal of bodily fluids from the chamber of the fluid collection assembly. In the latter case, a user may receive the indication (*e.g*., from the controller) and activate the vacuum pump manually.

The method 500 may include collecting the bodily fluids that are removed from the fluid collection assembly, such as into a fluid storage container that is spaced from the fluid collection assembly and fluidly coupled to the conduit. The fluid storage container may include any of the fluid storage containers disclosed herein.

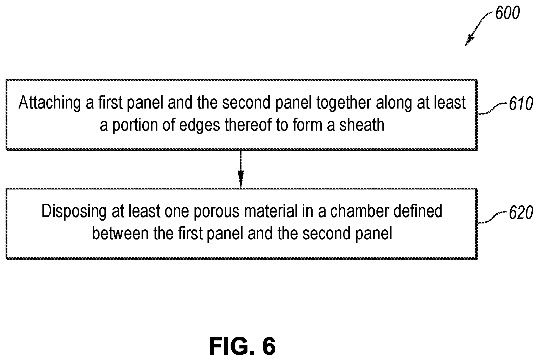

**FIG. 6** is a flow diagram of a method 600 to manufacture a fluid collection assembly not forming part of the invention and present for background information only. The method 600 may be used to manufacture at least some of the fluid collection assemblies and/or fluid collection systems disclosed herein. The method 600 may include act 610, which recites "attaching a first panel and the second panel together along at least a portion of edges thereof to form a sheath." Act 610 may be followed by act 620, which recites "disposing at least one porous material in a chamber defined between the first panel and the second panel."

Acts 610, 620 of the method 600 are for illustrative purposes. For example, the acts 610, 620 of the method 600 may be performed in different orders, split into multiple acts, modified, supplemented, or combined. In an example, one or more of the acts 610, 620 of the method 600 may be omitted from the method 600. Any of the acts 610 or 620 may include using any of the fluid collection devices or systems disclosed herein.

Act 610 recites "attaching at least a portion of a first panel and at least a portion of the second panel together along at least a portion of edges thereof to form a sheath." In an example, when the first and second panels are distinct sheets, act 610 may include positioning the first panel and the second panel adjacent to each other (*e.g*., on top of each other). Positioning the first panel and the second panel adjacent to each other forms a chamber between the first panel and the second panel. After positioning the first and second panels, the first and second panels may be attached to each other, such as attached to each other along at least a portion of the outer edges thereof. For instance, all of the outer edges of the first and second panels may be attached together except for portions of the outer edges thereof that define an opening configured to receive a penis (if the opening is not completely defined by one of the first or second panels) and a fluid outlet. In an example, the first and second panels are integrally formed together. In such an example, act 610 may include attaching edges of the first and second panels that are not already attached. When the first and second panels are formed by a thin walled tube, act 610 may include attaching the portions of the first and second panels that form the proximal and distal end regions thereof together. When the first and second panels are formed by a single folded sheet, act 610 may include attaching the portions of the first and second panels that form the proximal and distal end regions thereof together and attaching the portions of the first and second panels that are opposite the fold.

The first and second panels may be attached together using any suitable technique. In an embodiment, the first and second panels are attached together by sewing the first and second panels together. In an embodiment, the first and second panels are attached together using a non-sewing technique, such as via heat sealing, RF welding, or US welding. Using a non-sewing technique to attach the first and second panels together may increase the rate of manufacture of the fluid collection assembly formed during the method 600. For example, heat sealing, RF welding, or US welding may be performed significantly faster than sewing the first and second panels together. Further, heat sealing, RF welding, or US welding may form better water proof seals compared to sewing.

Act 620 recites "disposing at least one porous material in a chamber defined between the first panel and the second panel." In an embodiment, the porous material may be attached to one of the first or second panels (*e.g.*, with an adhesive, heat sealing, RF welding, US welding, or any other suitable technique) before act 610. In such an embodiment, positioning the first and second panels adjacent to each other also disposes the porous material in the chamber. In an embodiment, the porous material may be positioned between the first and second panels when the first and second panels are positioned adjacent to each other. In other words, the first panel, the porous material, and the second panel may form a stack with the porous material positioned between the first and second panels. Act 620 may also attach the porous materials to the first and second panels as the first and second panels are attached together. As such, the method 600 does not require a separate act of attaching the porous material to the first and second panels thus making the method 600 more efficient and quicker.

The porous material is disposed in the chamber after attaching the first and second panels together. In an example, as previously discussed, the porous material is attached to the first and second panel by attaching portions of the fluid impermeable barrier that are adjacent to one or more base gaps and one or more porous material gaps. In an example, the porous material may be attached to one or more of the first or second panels with an adhesive, tape, or the like or the porous material may not be attached to the first and second panels. While not attaching the porous material to the first and second panels may make the method 600 more efficient and quicker, the porous material may move in the chamber, such as by bunching up near the fluid outlet.

The method 600 may include forming one or more of the first panel, the second panel, and the porous material. In an example, the method 600 may form the first and second panel, for example, by stamping the first and second panels (including any openings, cutouts, or perforation defined thereby). Stamping the first and second panels and attaching the first and second panels together, as discussed above, may be quicker and easier than forming a single sheet into a cylinder. In an example, the porous material may also be stamped from a sheet of the porous material when the porous material exhibits a sheet-like shape. In an example, the first and second panel may be formed using a blown film extrusion technique to form a thin walled tube. In an example, at least one of the first panel, the second panel, or the porous material may be formed by non-stamping techniques or non-blown film extrusion technique even though such techniques may be more complicated or time consuming. Examples of non-stamping and non-blown film extrusion techniques include cutting with a blade, other extrusion techniques, molding (*e.g*., cast molding), or any other suitable technique.

The method 600 may include forming the base. In an example, the base may be formed by stamping the base from a sheet (*e*.*g*., a sheet that includes a substrate, at least one adhesive layer, and at least one release liner). The base may be formed by stamping since, for instance, the base is formed from a thin film and the base may not include any protrusions, such as a ring-like protrusion defining the aperture. Alternatively, the base may be formed by injection molding, or any other suitable technique even though non-stamping techniques may be more time consuming.

The method 600 may include attaching (*e*.*g*., permanently attaching) the base to the sheath formed at least partially by acts 610 and 620. In an example, the method 600 includes attaching the base to the sheath before providing the fluid collection assembly to an end user. The method 600 may include permanently attaching the base to the sheath and, after attaching the base to the sheath, disposing the fluid collection assembly in a package. The method 600 does not include permanently attaching the base to the sheath before providing the fluid collection assembly to an end user. The method 600 may include disposing the fluid collection assembly, with the sheath and the base not attached together, in a package. The end user may remove the fluid collection assembly from the package and may permanently attaching the base to the sheath after removing the fluid collection assembly from the package. For instance, the end user may permanently attach the sheath to the base before, during, or after attaching the base to the skin surround the penis.

It is noted that the embodiments disclosed above relate to fluid collection assemblies configured to collection bodily fluids from a male. However, it is noted that such fluid collection assemblies may also be used to collection bodily fluids from a female since the urethral opening of the female is, functionally, similar to a buried penis.

Terms of degree (*e*.*g*., "about," "substantially," "generally," etc.) indicate structurally or functionally insignificant variations. In an example, when the term of degree is included with a term indicating quantity, the term of degree is interpreted to mean ± 10%, ±5%, or +2% of the term indicating quantity. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc.

## Claims

1. An assembly (100) for collecting fluid from a penis, the assembly,
comprising:
a sheath (102) including:
a fluid impermeable barrier (106) which includes a first panel (108) and a second panel (110) attached to the first panel, the barrier including a proximal region (160) and a distal region (162) extending from the proximal region, the proximal region exhibiting a first width and the distal region exhibiting a second width that is less than the first width, the proximal region defining an opening (114) in the second panel for the penis, the fluid impermeable barrier at least defining a chamber (112) and the distal region defining a fluid outlet (118) for fluid in the chamber, the fluid outlet being formed from a portion of the first panel (108) and the second panel (110) that are not attached together; and
at least one porous material (122) disposed in the chamber; and
a base (104) secured to or configured to be secured to the proximal region of the sheath, the base configured to be attached to skin surrounding a penis, the base defining an aperture (124) that corresponds to the opening of the sheath, and wherein the sheath further includes a port (130) attached to the fluid outlet, the port including a first part (144) defining an inlet (148) and a second part (146) defining a port outlet (150), with the first part disposed between the first and second panels, the port including a channel communicating between the chamber and the port outlet, the second part configured to be attached to a conduit, wherein:
the first part exhibits a first maximum thickness and
the second part exhibits a second maximum thickness that is the same or greater than the first maximum thickness; and
the first part exhibits a first width and the second part exhibits a second width,
**characterized in that**
the first width is at least two times greater than the second width.

2. The fluid collection assembly of claim 1, wherein the first panel and the second panel are integrally formed together.

3. The fluid collection assembly of claim 1 or 2, wherein the fluid impermeable barrier includes polyurethane.

4. The fluid collection assembly of any one of the preceding claims, wherein the first width is substantially constant and the second width decreases from the proximal region towards the fluid outlet.

5. The fluid collection assembly of any one of the preceding claims, wherein:
the fluid impermeable barrier defines one or more orifices (132) extending therethrough; and
the sheath further includes one or more vents (134) attached to the fluid impermeable barrier and covering the one or more orifices, the one or more vents configured to allow air to flow therethrough and substantially prevent water flowing therethrough.

6. The fluid collection assembly of claim 5, wherein the one or more orifices and the one or more vents exhibits an elongated shape.

7. The fluid collection assembly of any one of the preceding claims, wherein the first part defines a sink upstream from the inlet, the sink exhibiting at least one of a width or thickness that is greater than a corresponding width or thickness of the inlet.

8. The fluid collection assembly of any one of the preceding claims, in which the first part exhibits a generally diamond cross-sectional shape.

9. The fluid collection assembly of any one of the preceding claims, wherein the port includes at least one tab (158) extending from the first part into the chamber.

10. The fluid collection assembly of any one of the preceding claims, wherein the base includes a nonwoven substrate.

11. The fluid collection assembly of any one of the preceding claims, wherein the base defines one or more base gaps (196) therein; and
the at least one porous material defines one or more porous material gaps (198) extending therethrough, wherein the one or more porous material gaps are generally aligned with the one or more base gaps; and
wherein opposing portions of the fluid impermeable barrier that are generally aligned with the one or more base gaps and the one or more porous material gaps are attached together.

12. The fluid collection assembly of any one of the preceding claims, wherein the base includes an inner region adjacent to the aperture and an outer region spaced from the aperture by the inner region, and wherein the inner region is attached to the fluid impermeable barrier and the outer region is not attached to the fluid impermeable barrier.

13. A system (407), comprising:
the fluid collection assembly of any one of the preceding claims:
a vacuum source (411) configured to apply a vacuum force;
a fluid storage container (409); and
at least one conduit (142) connected to the port outlet and in fluid communication with the vacuum source and the fluid storage container.

## Patentansprüche

1. Anordnung (100) zum Sammeln von Flüssigkeit von einem Penis, wobei die Anordnung Folgendes umfasst:
eine Hülle (102), einschließend:
eine fluidundurchlässige Barriere (106), die eine erste Platte (108) und eine an der ersten Platte befestigte zweite Platte (110) einschließt, wobei die Barriere einen proximalen Bereich (160) und einen distalen Bereich (162) einschließt, der sich von dem proximalen Bereich aus erstreckt, wobei der proximale Bereich eine erste Breite aufweist und der distale Bereich eine zweite Breite aufweist, die geringer ist als die erste Breite, wobei der proximale Bereich eine Aussparung (114) in der zweiten Platte für den Penis definiert, wobei die fluidundurchlässige Barriere mindestens eine Kammer (112) definiert und der distale Bereich einen Fluidauslass (118) für Fluid in der Kammer definiert, wobei der Fluidauslass aus einem Abschnitt der ersten Platte (108) und der zweiten Platte (110) gebildet ist, die nicht miteinander verbunden sind; und
mindestens ein poröses Material (122), das in der Kammer angeordnet ist; und
eine Basis (104), die an dem proximalen Bereich der Hülle befestigt oder so ausgebildet ist, dass sie daran befestigt werden kann, wobei die Basis so ausgebildet ist, dass sie an der einen Penis umgebenden Haut angebracht werden kann, wobei die Basis eine Öffnung (124) definiert, die der Aussparung der Hülle entspricht, und wobei die Hülle weiter einen Anschluss (130) einschließt, der an dem Fluidauslass angebracht ist, wobei der Anschluss einen ersten Teil (144), der einen Einlass (148) definiert, und einen zweiten Teil (146), der einen Anschlussauslass (150) definiert, einschließt, wobei der erste Teil zwischen der ersten und der zweiten Platte angeordnet ist, wobei der Anschluss einen Kanal einschließt, der eine Verbindung zwischen der Kammer und dem Anschlussauslass herstellt, wobei der zweite Teil so ausgebildet ist, dass er an einer Leitung angebracht werden kann, wobei:
der erste Teil eine erste maximale Dicke aufweist und
der zweite Teil eine zweite maximale Dicke aufweist, die gleich oder größer ist als die erste maximale Dicke; und
der erste Teil eine erste Breite aufweist und der zweite Teil eine zweite Breite aufweist,
**dadurch gekennzeichnet, dass**
die erste Breite mindestens zweimal so groß ist wie die zweite Breite.

2. Fluidsammelanordnung nach Anspruch 1, wobei die erste Platte und die zweite Platte einstückig miteinander gebildet sind.

3. Fluidsammelanordnung nach Anspruch 1 oder 2, wobei die fluidundurchlässige Barriere Polyurethan einschließt.

4. Fluidsammelanordnung nach einem der vorstehenden Ansprüche, wobei die erste Breite im Wesentlichen konstant ist und die zweite Breite vom proximalen Bereich in Richtung des Fluidauslasses abnimmt.

5. Fluidsammelanordnung nach einem der vorstehenden Ansprüche, wobei:
die fluidundurchlässige Barriere einen oder mehrere Durchlässe (132) definiert, die sich durch sie hindurch erstrecken; und
die Hülle weiter eine oder mehrere Belüftungsöffnungen (134) einschließt, die an der fluidundurchlässigen Barriere angebracht sind und den einen oder die mehreren Durchlässe abdecken, wobei die eine oder mehreren Belüftungsöffnungen so ausgebildet sind, dass sie Luft durch sie hindurchströmen lassen und im Wesentlichen verhindern, dass Wasser durch sie hindurchfließt.

6. Fluidsammelanordnung nach Anspruch 5, wobei der eine oder die mehreren Durchlässe und die eine oder mehrere Belüftungsöffnungen eine längliche Form aufweisen.

7. Fluidsammelanordnung nach einem der vorstehenden Ansprüche, wobei der erste Teil eine Vertiefung stromaufwärts vom Einlass definiert, wobei die Vertiefung mindestens eines von einer Breite oder Dicke aufweist, die größer als eine entsprechende Breite oder Dicke des Einlasses ist.

8. Fluidsammelanordnung nach einem der vorstehenden Ansprüche, wobei der erste Teil eine allgemein rautenförmige Querschnittsform aufweist.

9. Fluidsammelanordnung nach einem der vorstehenden Ansprüche, wobei der Anschluss mindestens eine Lasche (158) einschließt, die sich von dem ersten Teil in die Kammer erstreckt.

10. Fluidsammelanordnung nach einem der vorstehenden Ansprüche, wobei die Basis ein Vliesstoffsubstrat einschließt.

11. Fluidsammelanordnung nach einem der vorstehenden Ansprüche, wobei die Basis eine oder mehrere Basislücken (196) darin definiert; und
das mindestens eine poröse Material eine oder mehrere Lücken des porösen Materials (198) definiert, die sich durch dieses hindurch erstrecken, wobei die eine oder mehreren Lücken des porösen Materials im Allgemeinen mit der einen oder den mehreren Basislücken ausgerichtet sind; und
wobei gegenüberliegende Abschnitte der fluidundurchlässigen Barriere, die im Allgemeinen mit dem einen oder den mehreren Basislücken und dem einen oder den mehreren Lücken des porösen Materials ausgerichtet sind, miteinander verbunden sind.

12. Fluidsammelanordnung nach einem der vorstehenden Ansprüche, wobei die Basis einen an die Öffnung angrenzenden inneren Bereich und einen durch den inneren Bereich von der Öffnung beabstandeten äußeren Bereich einschließt, und wobei der innere Bereich an der fluidundurchlässigen Barriere angebracht ist und der äußere Bereich nicht an der fluidundurchlässigen Barriere angebracht ist.

13. System (407), umfassend:
die Fluidsammelanordnung nach einem der vorstehenden Ansprüche:
eine Vakuumquelle (411), die so ausgebildet ist, dass sie eine Vakuumkraft ausübt;
einen Fluidspeicherbehälter (409); und
mindestens eine Leitung (142), die mit dem Anschlussauslass verbunden ist und in Fluidverbindung mit der Vakuumquelle und dem Fluidspeicherbehälter steht.

## Revendications

1. Ensemble (100) pour collecter du fluide à partir d'un pénis, l'ensemble comprenant :
une gaine (102) incluant :
une barrière (106) imperméable aux fluides qui inclut un premier panneau (108) et un second panneau (110) fixé au premier panneau, la barrière incluant une région proximale (160) et une région distale (162) s'étendant à partir de la région proximale, la région proximale présentant une première largeur et la région distale présentant une seconde largeur qui est inférieure à la première largeur, la région proximale définissant une ouverture (114) dans le second panneau pour le pénis, la barrière imperméable aux fluides définissant au moins une chambre (112) et la région distale définissant une sortie (118) de fluide pour le fluide dans la chambre, la sortie de fluide étant formée à partir d'une portion du premier panneau (108) et du second panneau (110) qui ne sont pas fixés ensemble ; et
au moins un matériau poreux (122) disposé dans la chambre ; et
une base (104) fixée solidement à ou configurée pour être fixée solidement à la région proximale de la gaine, la base étant configurée pour être fixée à la peau entourant un pénis, la base définissant une ouverture (124) qui correspond à l'ouverture de la gaine, et dans lequel la gaine inclut en outre un orifice (130) fixé à la sortie de fluide, l'orifice incluant une première partie (144) définissant une entrée (148) et une seconde partie (146) définissant une sortie (150) d'orifice, avec la première partie disposée entre les premier et second panneaux, l'orifice incluant un canal communiquant entre la chambre et la sortie d'orifice, la seconde partie configurée pour être fixée à un conduit, dans lequel :
la première partie présente une première épaisseur maximale et
la seconde partie présente une seconde épaisseur maximale qui est égale ou supérieure à la première épaisseur maximale ; et
la première partie présente une première largeur et la seconde partie présente une seconde largeur,
**caractérisé en ce que**
la première largeur est au moins deux fois supérieure à la seconde largeur.

2. Ensemble de collecte de fluide selon la revendication 1, dans lequel le premier panneau et le second panneau sont formés ensemble d'un seul tenant.

3. Ensemble de collecte de fluide selon la revendication 1 ou la revendication 2, dans lequel la barrière imperméable aux fluides inclut du polyuréthane.

4. Ensemble de collecte de fluide selon l'une quelconque des revendications précédentes, dans lequel la première largeur est sensiblement constante et la seconde largeur diminue à partir de la région proximale vers la sortie de fluide.

5. Ensemble de collecte de fluide selon l'une quelconque des revendications précédentes, dans lequel :
la barrière imperméable aux fluides définit un ou plusieurs orifices (132) s'étendant à travers celle-ci ; et
la gaine inclut en outre un ou plusieurs évents (134) fixés à la barrière imperméable aux fluides et recouvrant les un ou plusieurs orifices, les un ou plusieurs évents configurés pour permettre à l'air de s'écouler à travers ceux-ci et d'empêcher sensiblement l'eau de s'écouler à travers ceux-ci.

6. Ensemble de collecte de fluide selon la revendication 5, dans lequel les un ou plusieurs orifices et les un ou plusieurs évents présentent une forme allongée.

7. Ensemble de collecte de fluide selon l'une quelconque des revendications précédentes, dans lequel la première partie définit un piège en amont de l'entrée, le piège présentant au moins l'une d'une largeur ou d'une épaisseur qui est supérieure à une largeur ou une épaisseur correspondante de l'entrée.

8. Ensemble de collecte de fluide selon l'une quelconque des revendications précédentes, dans lequel la première partie présente une forme de section transversale généralement en diamant.

9. Ensemble de collecte de fluide selon l'une quelconque des revendications précédentes, dans lequel l'orifice inclut au moins une patte (158) s'étendant à partir de la première partie dans la chambre.

10. Ensemble de collecte de fluide selon l'une quelconque des revendications précédentes, dans lequel la base inclut un substrat non tissé.

11. Ensemble de collecte de fluide selon l'une quelconque des revendications précédentes, dans lequel la base définit un ou plusieurs espaces (196) de base à l'intérieur de celle-ci ; et
le au moins un matériau poreux définit un ou plusieurs espaces (198) de matériau poreux s'étendant à travers celui-ci, dans lequel les un ou plusieurs espaces de matériau poreux sont généralement alignés avec les un ou plusieurs espaces de base ; et
dans lequel les portions opposées de la barrière imperméable aux fluides qui sont généralement alignées avec les un ou plusieurs espaces de base et les un ou plusieurs espaces de matériau poreux sont fixées ensemble.

12. Ensemble de collecte de fluide selon l'une quelconque des revendications précédentes, dans lequel la base inclut une région interne adjacente à l'ouverture et une région externe espacée de l'ouverture par la région interne, et dans lequel la région interne est fixée à la barrière imperméable aux fluides et la région externe n'est pas fixée à la barrière imperméable aux fluides.

13. Système (407), comprenant :
l'ensemble de collecte de fluide selon l'une quelconque des revendications précédentes :
une source (411) de vide configurée pour appliquer une force de vide ;
un récipient (409) de stockage de fluide ; et
au moins un conduit (142) raccordé à la sortie d'orifice et en communication fluidique avec la source de vide et le récipient de stockage de fluide.
